(19) Europäisches Patentamt
European Patent Office
Office européen des brevets

(11) Veröffentlichungsnummer: **0 354 329 B1**

(12) # EUROPÄISCHE PATENTSCHRIFT

(45) Veröffentlichungstag der Patentschrift: **10.02.93**

(21) Anmeldenummer: **89111520.6**

(22) Anmeldetag: **24.06.89**

(51) Int. Cl.5: **C07C 309/35**, A01N 41/04, C07C 309/87, C07C 323/21, C07C 311/29, C07D 231/12, C07D 233/56, C07C 313/00, C07C 311/51

(54) **(Hetero)Aryloxynaphthaline mit über Schwefel gebundenen Substituenten.**

(30) Priorität: **09.07.88 DE 3823318**

(43) Veröffentlichungstag der Anmeldung: **14.02.90 Patentblatt 90/07**

(45) Bekanntmachung des Hinweises auf die Patenterteilung: **10.02.93 Patentblatt 93/06**

(84) Benannte Vertragsstaaten: **BE CH DE FR GB IT LI NL**

(56) Entgegenhaltungen:
**EP-A- 0 179 015**
**DE-A- 2 366 040**
**DE-A- 2 520 815**
**US-A- 4 521 426**

(73) Patentinhaber: **BAYER AG**

**W-5090 Leverkusen 1 Bayerwerk(DE)**

(72) Erfinder: **Andree, Roland, Dr.**
**Schillerstrasse 17**
**W-4018 Langenfeld(DE)**
Erfinder: **Haug, Michael, Dr.**
**Fahner Weg 5**
**W-5060 Bergisch-Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch-Gladbach 2(DE)**
Erfinder: **Strang, Harry, Dr.**
**Unterdorfstrasse 6A**
**W-4000 Düsseldorf 31(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 354 329 B1

## Beschreibung

Die Erfindung betrifft neue (Hetero)Aryloxynaphthaline mit über Schwefel gebundenen Substituenten, Verfahren und neue Zwischenprodukte zu ihrer Herstellung sowie ihre Verwendung als Herbizide.

Es ist bereits bekannt, daß bestimmte Dioxy-benzol-Derivate, wie z. B. α-[4-(2,4-Dichlor-phenoxy)-phenoxyl-propionsäure-methylester (Diclofop-methyl), herbizid wirksam sind (vgl. DE-OS 22 23 894 und DE-OS-25 20 815 und DE-OS-23 66 040). Die Wirkung dieser bekannten Verbindungen gegen Unkräuter sowie ihre Kulturpflanzen-Verträglichkeit sind jedoch nicht immer zufriedenstellend.

Es wurden nun neue (Hetero)Aryloxynaphthaline mit über Schwefel gebundenen Substituenten der allgemeinen Formel (I)

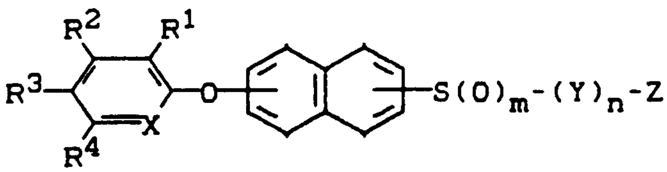

(I)

in welcher

- m für die Zahlen 0, 1 oder 2 steht,
- n für die Zahlen 0 oder 1 steht,
- $R^1$ für Wasserstoff, Halogen, Cyano oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- X für Stickstoff oder die Gruppierung C-$R^5$ steht, worin
- $R^5$ für Wasserstoff oder Halogen steht,
- Y für Sauerstoff oder eine der Gruppierungen

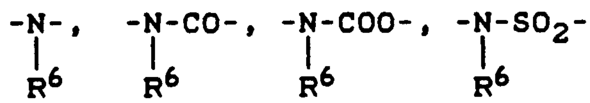

steht,
worin

- $R^6$ für Wasserstoff, Alkyl, -CO-Z oder -$SO_2$-Z steht und
- Z für Wasserstoff, Halogen oder einen gegebenenfalls substituierten Rest aus der Reihe Alkyl, Cycloalkyl, Cycloalkylalkyl, Alkenyl, Alkinyl, Aralkyl, Aryl und Heteroaryl steht,

sowie Salze von Verbindungen der Formel (I) (z.B. mit Z = Metalläquivalent) gefunden.

Weiter wurde gefunden, daß man die neuen (Hetero)Aryloxynaphthaline mit über Schwefel gebundenen Substituenten der allgemeinen Formel (I) erhält, wenn man

(a) für den Fall, daß m für 2 steht, n für 1 steht, Y für Sauerstoff steht, Z für Wasserstoff oder ein Metalläquivalent steht und $R^1$, $R^2$, $R^3$, $R^4$ und X die oben angegebenen Bedeutungen haben, Halogen-(hetero)aryl-Verbindungen der allgemeinen Formel (II)

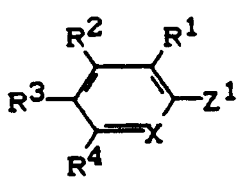

(II)

in welcher

$R^1$, $R^2$, $R^3$, $R^4$ und X die oben angegebenen Bedeutungen haben und

$Z^1$ für Halogen steht,
mit Hydroxynaphthalinsulfonsäuren der allgemeinen Formel (III)

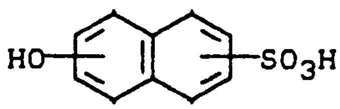

(III)

oder deren Metallsalzen
gegebenenfalls in Gegenwart eines Säureakzeptors und gegebenenfalls in Gegenwart eines Verdünnungsmittels umsetzt und gegebenenfalls anschließend mit einer starken Säure behandelt, oder wenn man
(b) für den Fall, daß m für die Zahlen 0, 1 oder 2 steht, n für die Zahl 0 steht, Z für Halogen steht und $R^1$, $R^2$, $R^3$, $R^4$ und X die oben angegebenen Bedeutungen haben,
Verbindungen der allgemeinen Formel (I), in welcher m für die Zahlen 0, 1 oder 2 steht, n für die Zahl 1 steht, Y für Sauerstoff steht, Z für Wasserstoff oder ein Metalläquivalent steht und $R^1$, $R^2$, $R^3$, $R^4$ und X die oben angegebenen Bedeutungen haben,
mit einem Halogenierungsmittel, gegebenenfalls in Gegenwart eines Katalysators bzw. Halogenierungshilfsmittels, und gegebenenfalls in Gegenwart eines Verdünnungsmittels umsetzt, oder wenn man
(c) für den Fall, daß m für die Zahlen 0,1 oder 2 steht, n für die Zahl 0 steht, Z für Wasserstoff oder ein Metalläquivalent steht und $R^1$, $R^2$, $R^3$, $R^4$ und X die oben angegebenen Bedeutungen haben,
Verbindungen der allgemeinen Formel (I), in welcher m für die Zahl 2 steht, n für die Zahl 0 steht, Z für Halogen steht und $R^1$, $R^2$, $R^3$, $R^4$ und X die oben angegebenen Bedeutungen haben,
mit einem Reduktionsmittel, gegebenenfalls in Gegenwart eines Reduktionshilfsmittels, und gegebenenfalls in Gegenwart eines Verdünnungsmittels umsetzt, oder wenn man
(d) für den Fall, daß m für die Zahlen 0, 1 oder 2 steht, n für die Zahl 1 steht und $R^1$, $R^2$, $R^3$, $R^4$, X, Y und Z die oben angegebenen Bedeutungen haben,
Verbindungen der Formel (I), in welcher m für die Zahlen 0, 1 oder 2 steht, n für die Zahl 0 steht, Z für Halogen steht und $R^1$, $R^2$, $R^3$, $R^4$ und X die oben angegebenen Bedeutungen haben,
mit nucleophilen Verbindungen der allgemeinen Formel (IV)

$M-(Y)_n-Z$ (IV)

in welcher
n, Y und Z die oben angegebenen Bedeutungen haben und
M für Wasserstoff oder ein Metalläquivalent steht,
gegebenenfalls in Gegenwart eines Säureakzeptors und gegebenenfalls in Gegenwart eines Verdünnungsmittels umsetzt, oder wenn man
(e) für den Fall, daß Z mit Ausnahme von Wasserstoff und Halogen die oben angegebene Bedeutung hat und m, n, $R^1$, $R^2$, $R^3$, $R^4$, X und Y die oben angegebenen Bedeutungen haben,
Verbindungen der Formel (I) in welcher Y für Sauerstoff oder NH steht, Z für Wasserstoff steht und m, n, $R^1$, $R^2$, $R^3$, $R^4$ und X die oben angegebenen Bedeutungen haben,
mit elektrophilen Verbindungen der allgemeinen Formel (V)

$Z^2-(Y^1)_n-Z^3$ (V)

in welcher
n für die Zahlen 0 oder 1 steht,
$Y^1$ für CO oder $SO_2$ steht,
$Z^2$ für Halogen steht und
$Z^3$ mit Ausnahme von Wasserstoff und Halogen die oben für Z angegebene Bedeutung hat,
gegebenenfalls in Gegenwart eines Säureakzeptors und gegebenenfalls in Gegenwart eines Verdünnungsmittels umsetzt
und gegebenenfalls aus den nach den oben beschriebenen Verfahren hergestellten Verbindungen der Formel (I) Salze nach üblichen Methoden herstellt.

Schließlich wurde gefunden, daß die neuen (Hetero)Aryloxynaphthaline mit über Schwefel gebundenen Substituenten der Formel (I) hervorragende herbizide Eigenschaften aufweisen.

Überraschenderweise sind die erfindungsgemäßen (Hetero)-Aryloxynaphthaline mit über Schwefel gebundenen Substituenten der Formel (I) gegen Unkräuter wesentlich stärker wirksam als α-[4-(2,4-Dichlorphenoxy)-phenoxy]-propionsäure-methylester, welches ein strukturell ähnlicher vorbekannter Wirkstoff gleicher Wirkungsrichtung ist.

Die Erfindung betrifft vorzugsweise Verbindungen der Formel (I), in welcher

m für die Zahlen 0, 1 oder 2 steht,

n für die Zahlen 0 oder 1 steht,

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff Fluor oder Chlor steht,

X für Stickstoff oder die Gruppierung C-$R^5$ steht, worin

$R^5$ für Wasserstoff, Fluor, Chlor oder Brom steht,

Y für Sauerstoff oder eine der Gruppierungen

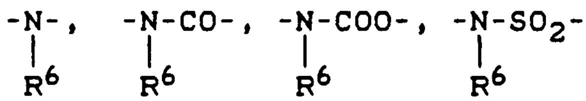

steht,

worin

$R^6$ für Wasserstoff, $C_1$-$C_4$-Alkyl, -CO-Z oder -$SO_2$-Z steht und

Z für Wasserstoff, Halogen, für gegebenenfalls durch Halogen, Cyano, Carboxy, $C_1$-$C_4$-Alkoxy, $C_1$-$C_4$-Alkyl-carbonyl, $C_1$-$C_4$-Alkoxy-carbonyl, $C_1$-$C_4$-Alkoxy-carbonyl-$C_1$-$C_4$-alkoxy-carbonyl und/oder $C_1$-$C_4$-Alkylamino-carbonyl substituiertes $C_1$-$C_6$-Alkyl, für jeweils gegebenenfalls durch Halogen und/oder $C_1$-$C_4$-Alkyl substituiertes $C_3$-$C_6$-Cycloalkyl oder $C_3$-$C_6$-Cycloalkyl-$C_1$-$C_4$-alkyl, für jeweils gegebenenfalls durch Halogen, Cyano, Carboxy und/oder $C_1$-$C_4$-Alkoxy-carbonyl substituiertes $C_2$-$C_6$-Alkenyl oder $C_2$-$C_6$-Alkinyl, für jeweils gegebenenfalls durch Halogen, Cyano, Carboxy, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfinyl, $C_1$-$C_4$-Alkylsulfonyl, Di-($C_1$-$C_2$-alkyl)-aminosulfonyl, Di-($C_1$-$C_2$-alkyl)-aminocarbonyl, Di-($C_1$-$C_2$-alkyl)-amino, $C_1$-$C_4$-Alkoxy-carbonyl und/oder $C_1$-$C_2$-Alkylendioxy substituiertes Phenyl, Naphthyl, Phenyl-$C_1$-$C_4$-alkyl oder Naphthyl-$C_1$-$C_4$-alkyl steht, oder für einen gegebenenfalls durch Halogen, $C_1$-$C_4$-Alkyl, $C_1$-$C_2$-Halogenalkyl, $C_1$-$C_4$-Alkoxy und/oder $C_1$-$C_2$-Halogenalkoxy substituierten Heterocyclus aus der Reihe Pyrazolyl, Imidazolyl, Triazolyl steht.

Die Erfindung betrifft insbesondere Verbindungen der Formel (I), in welcher

$R^1$ für Cyano, Fluor oder Chlor steht,

$R^2$ für Wasserstoff, Fluor oder Chlor steht,

$R^3$ für Chlor, Trifluormethyl oder Trifluormethylsulfonyl steht,

$R^4$ für Wasserstoff, Fluor oder Chlor steht,

X für Stickstoff oder die Gruppierung C-$R^5$ steht, worin

$R^5$ für Wasserstoff, Fluor oder Chlor steht,

und

(A)

m für die Zahl 0 steht,

n für die Zahl 0 steht und

Z für Wasserstoff, für gegebenenfalls durch Fluor, Chlor, Cyano, Carboxy, $C_1$-$C_4$-Alkoxy-carbonyl, $C_1$-$C_4$-Alkoxy-carbonyl-$C_1$-$C_2$-alkoxy-carbonyl und/oder $C_1$-$C_4$-Alkylamino-carbonyl substituiertes $C_1$-$C_4$-Alkyl, für gegebenenfalls durch Fluor, Chlor, Cyano, Carboxy und/oder $C_1$-$C_4$-Alkoxy-carbonyl substituiertes $C_3$-$C_4$-Alkenyl oder für gegebenenfalls durch Fluor, Chlor und/oder Methyl substituiertes Benzyl steht, oder

(B)

m für die Zahl 2 steht,

n für die Zahlen 0 oder 1 steht,

Y für Sauerstoff oder eine der Gruppierungen -NH-, -NH-CO-, -NH-COO-, -NH-$SO_2$- steht, und

Z für Wasserstoff, für gegebenenfalls durch Fluor, Chlor, Cyano, Carboxy, $C_1$-$C_4$-Alkoxyccarbonyl und/oder $C_1$-$C_4$-Alkylamino-carbonyl substituiertes $C_1$-$C_4$-Alkyl, für gegebenenfalls durch Fluor, Chlor, Cyano, Carboxy und/oder $C_1$-$C_4$-Alkoxy-carbonyl substituiertes $C_3$-$C_4$-Alkenyl, für jeweils gegebenenfalls durch Fluor, Chlor, Brom, Nitro, Methyl, Ethyl, Trifluormethyl, Methoxy, Ethoxy, Difluormethoxy, Trifluormethoxy, Chlordifluormethoxy, Methylthio, Difluormethylthio, Trifluormethylthio, Chlordifluormethylthio, Methylsulfonyl, Dimethylaminosulfonyl, Methoxycarbonyl und/oder Ethoxycarbonyl substituiertes Phenyl oder Benzyl steht, oder

(C)

m für die Zahl 2 steht,

n für die Zahl 0 steht und

Z für Chlor, für gegebenenfalls durch Chlor und/oder $C_1$-$C_3$-Alkyl substituiertes Pyrazolyl oder für gegebenenfalls durch $C_1$-$C_3$-Alkyl substituiertes Imidazolyl steht.

Ganz besonders bevorzugte Gruppen von Verbindungen der Formel (I) sind diejenigen der nachstehenden Formeln

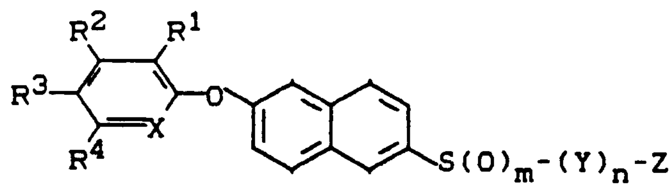

(Ia)

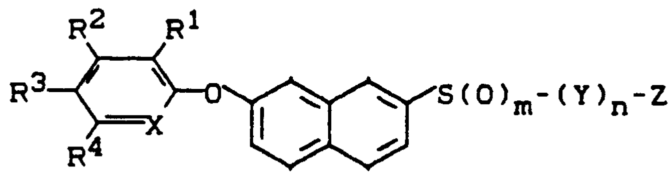

(Ib)

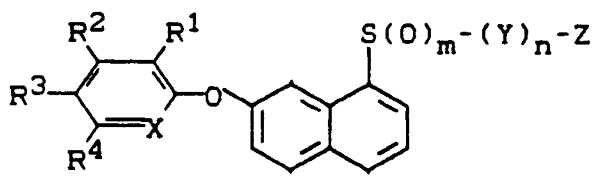

(Ic)

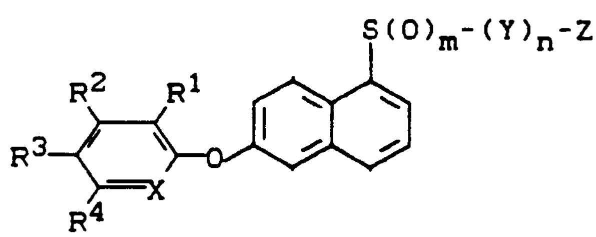

(Id)

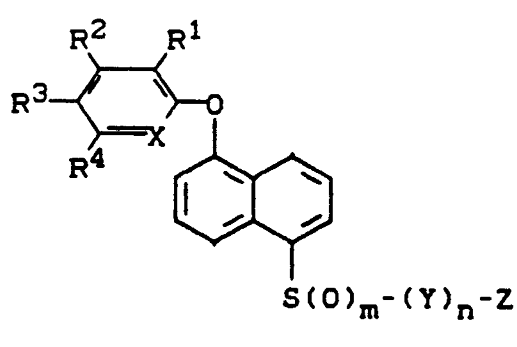

(Ie)

in welchen jeweils m, n, $R^1$, $R^2$, $R^3$, $R^4$, X, Y und Z die oben als insbesonders bevorzugt angegebenen

Bedeutungen haben.

Die Erfindung betrifft weiter vorzugsweise Salze aus Verbindungen der Formel (I), in welcher $R^1$, $R^2$, $R^3$, $R^4$, X, Y, Z, m und n die oben angegebenen Bedeutungen haben, soweit sie -COOH, $-SO_3H$, -CO-NH- oder $-SO_2$-HN-Gruppen enthalten, und Basen, wie vorzugsweise Natrium-, Kalium- oder Calciumhydroxid, -hydrid, -amid oder -carbonat, Natrium- oder Kalium-$C_1$-$C_4$-alkanolaten, Ammoniak, $C_1$-$C_4$-Alkylaminen, Di-($C_1$-$C_4$-alkyl)-aminen oder Tri-($C_1$-$C_4$-alkyl)-aminen.

Verwendet man für das erfindungsgemäße Verfahren (a) 3,4-Dichlor-benzotrifluorid und 7-Hydroxy-naphthalin-2-sulfonsäure-Kaliumsalz als Ausgangsstoffe, so kann der Reaktionsablauf durch das folgende Formelschema wiedergegebenen werden:

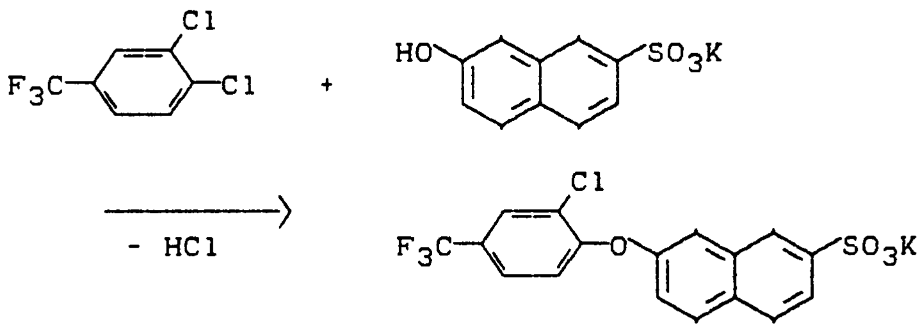


Verwendet man für das erfindungsgemäße Verfahren (b) 6-(2-Chlor-6-fluor-4-trifluormethyl-phenoxy)-naphthalin-2-sulfonsäure und Phosphor(V)-chlorid als Ausgangsstoffe, so kann Reaktionsablauf durch das folgende Formelschema wiedergegeben werden:

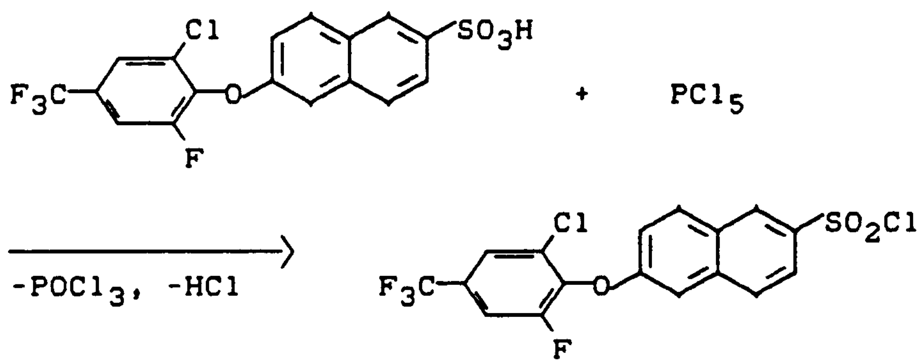


Verwendet man für das erfindungsgemäße Verfahren (c) 5-(2-Fluor-4-trifluormethyl-phenoxy)-naphthalin-1-sulfonsäurechlorid und Natriumsulfit als Ausgangsstoffe, so kann der Reaktionsablauf durch das folgende Formelschema wiedergegeben werden:

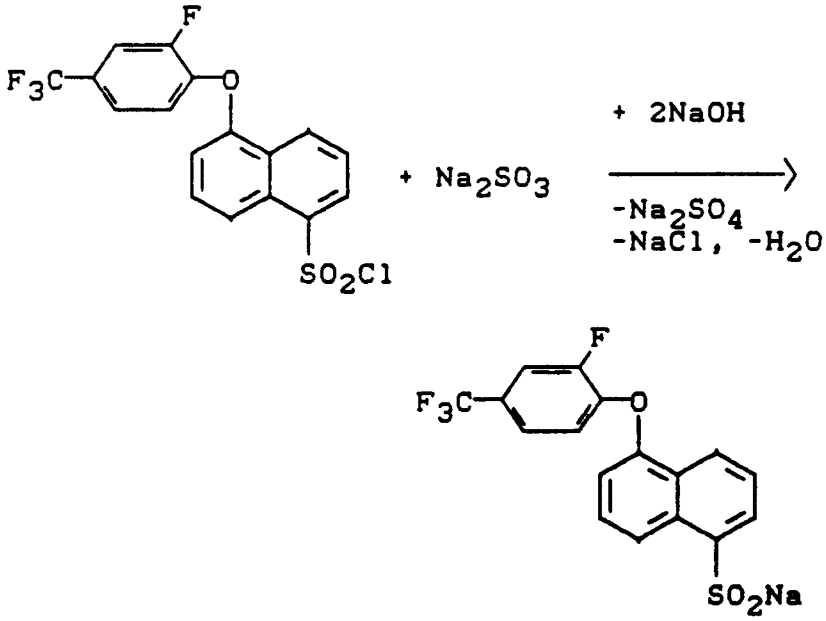


Verwendet man für das erfindungsgemäße Verfahren (d) 6-(3,5-Dichlor-pyridin-2-yl-oxy)-naphthalin-1-sulfonsäurechlorid und Aminoessigsäureethylester als Ausgangsstoffe, so kann der Reaktionsablauf durch das folgende Formelschema wiedergegeben werden:

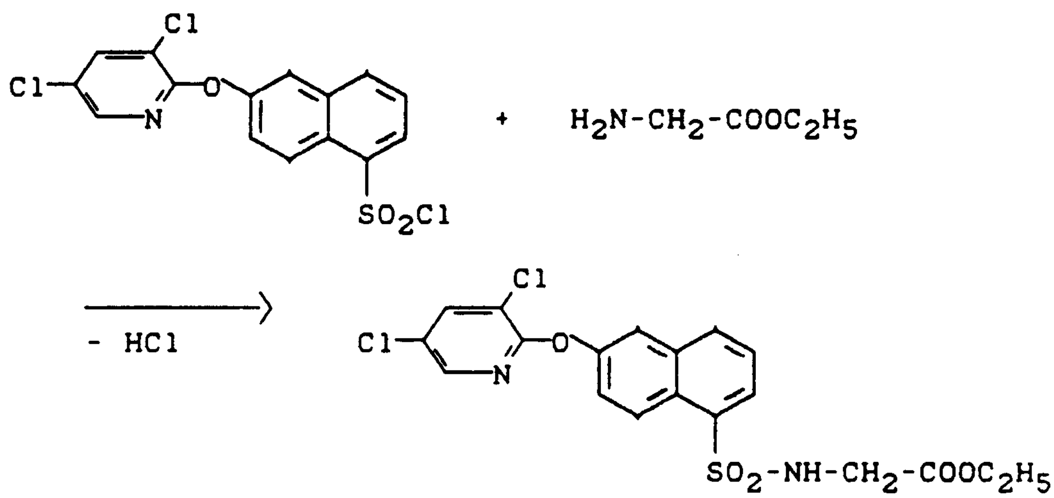


Verwendet man für das erfindungsgemäße Verfahren (e) 7-(3-Chlor-5-trifluormethyl-pyridin-2-yl-oxy)-naphthalin-1-yl-mercaptan und Chloressigsäureethylester als Ausgangsstoffe, so kann der Reaktionsablauf durch das folgende Formelschema wiedergegeben werden:

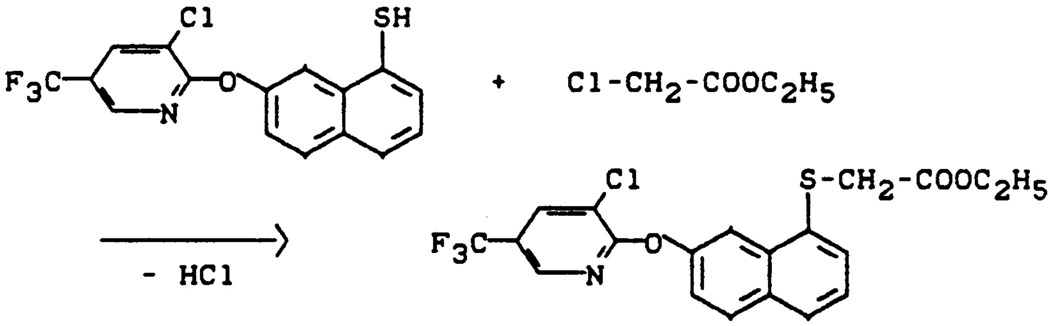


Die beim erfindungsgemäßen Verfahren (a) zur Herstellung von Verbindungen der Formel (I) als Ausgangsstoffe zu verwendenden Halogen-(hetero)aryl-Verbindungen sind durch die Formel (II) allgemein definiert.

In Formel (II) haben $R^1$, $R^2$, $R^3$, $R^4$ und X vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und X angegeben wurden und $Z^1$ steht vorzugsweise für Fluor oder Chlor.

Als Beispiele für die Ausgangsstoffe der Formel (II) seien genannt:
4-Chlor-benzotrifluorid, 3,4-Dichlor-benzotrifluorid, 3,4,5-Trichlor-benzotrifluorid, 3,4-Dichlor-5-fluor-benzotrifluorid, 2,3,4,5-Tetrachlor-benzotrifluorid, 3,5-Dichlor-2,4-difluor-benzotrifluorid, 3-Chlor-4,5-difluor-benzotrifluorid und 2,3,5-Trichlor-pyridin.

Die Verbindungen der Formel (II) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x)).

Als Beispiele für die bei Verfahren (a) weiter als Ausgangsstoffe zu verwendenden Hydroxynaphthalinsulfonsäuren der Formel (III) und ihre Salze seien gennant:
6-Hydroxy-naphthalin-2-sulfonsäure sowie deren Natrium-und Kalium-Salze, 7-Hydroxy-naphthalin-2-sulfonsäure sowie deren Natrium-und Kalium-Salze, 7-Hydroxy-naphthalin-1-sulfonsäuresowie deren Natrium-und Kalium-Salze, 6-Hydroxy-naphthalin-1-sulfonsäure sowie deren Natrium-und Kalium-Salze und 5-Hydroxy-naphthalin-1-sulfonsäure sowie deren Natrium-und Kalium-Salze.

Die Hydroxynaphthalinsulfonsäuren der Formel (III) sind bekannte organische Synthesechemikalien.

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran and Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Aprotische polare organische Lösungsmittel, wie z.B. Aceton, Acetonitril, Methylethylketon, Propionitril, Dimethylformamid, Dimethylacetamid, Dimethylsulfoxid, Sulfolan und N-Methylpyrrolidon werden besonders bevorzugt.

Als Säureakzeptoren können bei dem erfindungsgemäßen Verfahren (a) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0 °C und 200 °C, vorzugsweise bei Temperaturen zwischen 20 °C und 150 °C.

Das erfindungsgemäße Verfahren (a) wird im allgemeinen unter Normaldruck durchgeführt. Es ist

jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden.

Die bei Verfahren (a) im allgemeinen zunächst als Metallsalze anfallenden Produkte können durch Behandeln mit starken Säuren, wie z.B. Salzsäure oder Schwefelsäure, in die entsprechenden freien Säuren umgewandelt werden.

Die bei dem erfindungsgemäßen Verfahren (b) als Ausgangsstoffe zu verwendenden Verbindungen sind durch die Formel (I) mit der Maßgabe, daß n für die Zahl 1 steht, Y für Sauerstoff steht und Z für Wasserstoff oder ein Metalläquivalent steht, allgemein definiert. In diesem Fall haben m, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden und Z steht vorzugsweise für Wasserstoff, Natrium oder Kalium.

Beispiele für die Ausgangsstoffe zu Verfahren (b) sind in der nachstehenden Tabelle 1 aufgeführt.

Tabelle 1: Beispiele für die Ausgangsstoffe zu Verfahren (b)

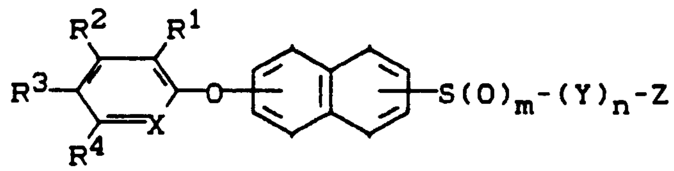

(I)

Hierbei gilt in jedem Einzelfall:
n = 1, Y = Sauerstoff

| m | $R^1$ | $R^2$ | $R^3$ | $R^4$ | X | Z |
|---|---|---|---|---|---|---|
| 0 | Cl | H | Cl | H | CH | H |
| 1 | Cl | H | Cl | H | CH | H |
| 2 | Cl | H | Cl | H | CH | H |
| 0 | Cl | H | Cl | H | N | H |
| 1 | Cl | H | Cl | H | N | H |
| 2 | Cl | H | Cl | H | N | H |
| 0 | Cl | H | $CF_3$ | H | CH | H |
| 1 | Cl | H | $CF_3$ | H | CH | H |
| 2 | Cl | H | $CF_3$ | H | CH | H |
| 0 | Cl | H | $CF_3$ | H | N | H |
| 1 | Cl | H | $CF_3$ | H | N | H |
| 2 | Cl | H | $CF_3$ | H | N | H |

Tabelle 1:- Fortsetzung

| m | $R^1$ | $R^2$ | $R^3$ | $R^4$ | X | Z |
|---|---|---|---|---|---|---|
| 0 | Cl | H | $CF_3$ | H | C-Cl | H |
| 1 | Cl | H | $CF_3$ | H | C-Cl | H |
| 2 | Cl | H | $CF_3$ | H | C-Cl | H |
| 0 | Cl | H | $CF_3$ | H | C-F | H |
| 1 | Cl | H | $CF_3$ | H | C-F | H |
| 2 | Cl | H | $CF_3$ | H | C-F | H |
| 0 | Cl | H | $CF_3$ | F | C-Cl | H |
| 1 | Cl | H | $CF_3$ | F | C-Cl | H |
| 2 | Cl | H | $CF_3$ | F | C-Cl | H |
| 0 | Cl | H | $CF_3$ | Cl | C-Cl | H |
| 1 | Cl | H | $CF_3$ | Cl | C-Cl | H |
| 2 | Cl | H | $CF_3$ | Cl | C-Cl | H |
| 0 | Cl | H | $SO_2CF_3$ | H | CH | H |
| 1 | Cl | H | $SO_2CF_3$ | H | CH | H |
| 2 | Cl | H | $SO_2CF_3$ | H | CH | H |
| 0 | Cl | H | $SO_2CF_3$ | H | C-Cl | H |
| 1 | Cl | H | $SO_2CF_3$ | H | C-Cl | H |
| 2 | Cl | H | $SO_2CF_3$ | H | C-Cl | H |
| 0 | Cl | H | $SO_2CF_3$ | H | C-F | H |
| 1 | Cl | H | $SO_2CF_3$ | H | C-F | H |
| 2 | Cl | H | $SO_2CF_3$ | H | C-F | H |
| 0 | CN | H | $CF_3$ | H | CH | H |
| 1 | CN | H | $CF_3$ | H | CH | H |
| 2 | CN | H | $CF_3$ | H | CH | H |

Die in der Tabelle 1 angegebenen Beispiele gelten im einzelnen jeweils für die ganz besonders bevorzugten Gruppen von Verbindungen der Formel (I), welche oben durch die Formeln (Ia), (Ib), (Ic), (Id) und (Ie) skizziert sind.

Die oben beschriebenen Ausgangsstoffe der Formel (I) für Verfahren (b) sind erfindungsgemäße, neue Verbindungen; sie können nach den erfindungsgemäßen Verfahren (a) oder (c) hergestellt werden.

Das erfindungsgemäße Verfahren (b) wird unter Einsatz eines Halogenierungsmittels durchgeführt. Für den Fall, daß m für die Zahl 0 steht, sind als Halogenierungsmittel insbesondere elementare Halogene, wie z.B. Chlor oder Brom, für den Fall, daß m für die Zahlen 1 oder 2 steht, ferner auch Säurehalogenide, wie z.B. Phosgen, Thionylchlorid, Chlorsulfonsäure, Phosphorylchlorid, Phosphor(III)-chlorid, Phosphor(III)-bromid und Phosphor(V)-chlorid, geeignet.

Als Katalysatoren bzw. Halogenierungshilfsmittel können zur Durchführung des erfindungsgemäßen Verfahrens (b) die üblicherweise zur Herstellung von Säurehalogeniden verwendeten Stoffe, wie z.B.

Pyridin, Dimethylformamid, Trimethylphosphinoxid, Triphenylphosphin oder auch Phosphoroxychlorid, eingesetzt werden.

Das erfindungsgemäße Verfahren (b) wird gegebenenfalls in Gegenwart eines Verdünnungsmittels durchgeführt. Vorzugsweise kommen relativ inerte organische Lösungsmittel aus der Reihe der halogenierten Kohlenwasserstoffe, wie z.B. Methylenchlorid, Chloroform, Tetrachlormethan, 1,2-Dichlor-ethan, Chlorbenzol oder 1,2-Dichlorbenzol, in Betracht.

Die Reaktionstemperaturen können bei dem erfindungsgemäßen Verfahren (b) in einem größeren Bereich variiert werden. Im allgemeinen arbeitet man bei Temperaturen zwischen 0 °C und 100 °C, vorzugsweise bei Temperaturen zwischen 10 °C und 90 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden je Mol Ausgangsverbindung der Formel (I) im allgemeinen zwischen 1 und 50 Mol, vorzugsweise zwischen 1,5 und 20 Mol, Halogenierungsmittel eingesetzt. Die Reaktionskomponenten werden im allgemeinen bei Raumtemperatur zusammengegeben und das Reaktionsgemisch wird, gegebenenfalls bei erhöhter Temperatur, bis zum Ende der Umsetzung gerührt. Die Reaktionsprodukte fallen bei Verfahren (b) im allgemeinen nach Einengen und Verdünnen des Rückstandes mit Wasser kristallin an und können durch Absaugen isoliert werden.

Die beim erfindungsgemäßen Verfahren (c) als Ausgangsstoffe zu verwendenden Verbindungen sind durch die Formel (I) mit der Maßgabe, daß m für die Zahl 2 steht, n für die Zahl 0 steht und Z für Halogen steht, allgemein definiert. In diesem Fall haben $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden und Z steht vorzugsweise für Chlor.

Beispiele für die Ausgangsstoffe zu verfahren (c) sind in der nachstehenden Tabelle 2 aufgeführt.

Tabelle 2: Beispiele für die Ausgangsstoffe zu Verfahren (c)

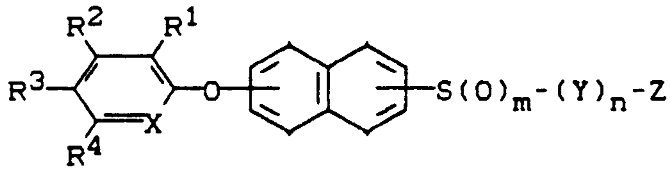

(I)

Hierbei gilt in jedem Einzelfall:

m = 2, n = 0

| $R^1$ | $R^2$ | $R^3$ | $R^4$ | X | Z |
|---|---|---|---|---|---|
| Cl | H | Cl | H | CH | Cl |
| Cl | H | Cl | H | N | Cl |
| Cl | H | $CF_3$ | H | CH | Cl |
| Cl | H | $CF_3$ | H | N | Cl |
| Cl | H | $CF_3$ | H | C-Cl | Cl |
| Cl | H | $CF_3$ | H | C-F | Cl |
| Cl | H | $CF_3$ | F | C-Cl | Cl |
| Cl | H | $CF_3$ | Cl | C-Cl | Cl |
| F | H | $CF_3$ | H | C-F | Cl |
| Cl | H | $SO_2CF_3$ | H | CH | Cl |
| Cl | H | $SO_2CF_3$ | H | C-Cl | Cl |
| CN | H | $CF_3$ | H | CH | Cl |

Die in Tabelle 2 angegebenen Beispiele gelten im einzelnen jeweils für die ganz besonders bevorzugten Gruppen von Verbindungen der Formel (I), welche oben durch die Formeln (Ia), (Ib), (Ic), (Id) und (Ie) skizziert sind.

Die oben beschriebenen Ausgangsstoffe der Formel (I) für Verfahren (c) sind erfindungsgemäße, neue Verbindungen; sie können nach dem erfindungsgemäßen Verfahren (b) hergestellt werden.

Das erfindungsgemäße Verfahren (c) wird unter Einsatz eines Reduktionsmittels durchgeführt. Als solche kommen die üblichen zur Reduktion von Sulfonsäurehalogeniden geeigneten Stoffe, wie z.B. Zink, Hydrogensulfid oder Natriumsulfit, in Betracht.

Als Reduktionshilfsmittel, welche in Abhängigkeit vom verwendeten Reduktionsmittel gegebenenfalls einzusetzen sind, seien Säuren, wie z.B. Salzsäure, Schwefelsäure und Essigsäure, bzw. Basen, wie z.B. Natronlauge und Kalilauge, genannt.

Das erfindungsgemäße Verfahren (c) wird vorzugsweise unter Verwendung von Verdünnungsmitteln durchgeführt. Als Verdünnungsmittel kommen neben Wasser praktische alle inerten organischen Lösungsmittel in Betracht, wie sie oben für das erfindungsgemäße Verfahren (a) angegeben sind. Besonders geeignet sind mit Wasser mischbare organische Lösungsmittel, wie z.B. Dioxan und Dimethylformamid.

Die Reaktionstemperaturen können bei dem erfindungsgemäßen Verfahren (c) in einem größeren Bereich variiert werden. Im allgemeinen arbeitet man bei Temperaturen zwischen 0 °C und 150 °C, vorzugsweise bei Temperaturen zwischen 10 °C und 120 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden je Mol Ausgangsverbindung der Formel (I) im allgemeinen zwischen 1 und 20 Mol, vorzugsweise zwischen 1 und 10 Mol, Reduktionsmittel eingesetzt. Die Reaktionskomponenten werden im allgemeinen bei Raumtemperatur oder bei geringfügig erhöhter oder erniedrigter Temperatur zusammengegeben und das Reaktionsgemisch wird, gegebenenfalls bei erhöhter Temperatur bis zum Ende der Umsetzung gerührt. Die Aufarbeitung kann nach üblichen Methoden durchgeführt werden.

Die beim erfindungsgemäßen Verfahren (d) als Ausgangsstoffe zu verwendenden Verbindungen sind durch die Formel (I) mit der Maßgabe, daß n für die Zahl 0 und Z für Halogen steht, allgemein definiert.
In diesem Fall haben m, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden und Z steht vorzugsweise für Chlor.

Beispiele für die Ausgangsstoffe der Formel (I) zu Verfahren (d) sind in der nachstehenden Tabelle 3 aufgeführt.

Tabelle 3: Beispiele für die Ausgangsstoffe zu Verfahren (d)

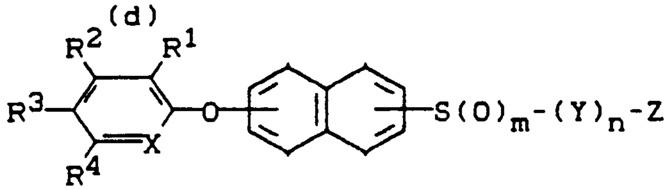
(I)

Hierbei gilt in jedem Einzelfall: n = 0

| m | $R^1$ | $R^2$ | $R^3$ | $R^4$ | X | Z |
|---|---|---|---|---|---|---|
| 0 | Cl | H | Cl | H | CH | Cl |
| 1 | Cl | H | Cl | H | CH | Cl |
| 2 | Cl | H | Cl | H | CH | Cl |
| 0 | Cl | H | Cl | H | N | Cl |
| 1 | Cl | H | Cl | H | N | Cl |
| 2 | Cl | H | Cl | H | N | Cl |
| 0 | Cl | H | $CF_3$ | H | CH | Cl |
| 1 | Cl | H | $CF_3$ | H | CH | Cl |
| 2 | Cl | H | $CF_3$ | H | CH | Cl |
| 0 | Cl | H | $CF_3$ | H | N | Cl |
| 1 | Cl | H | $CF_3$ | H | N | Cl |
| 2 | Cl | H | $CF_3$ | H | N | Cl |

Tabelle 3: - Fortsetzung

| m | $R^1$ | $R^2$ | $R^3$ | $R^4$ | X | Z |
|---|---|---|---|---|---|---|
| 0 | Cl | H | $CF_3$ | H | C-Cl | Cl |
| 1 | Cl | H | $CF_3$ | H | C-Cl | Cl |
| 2 | Cl | H | $CF_3$ | H | C-Cl | Cl |
| 2 | CN | H | $CF_3$ | H | CH | Cl |
| 0 | Cl | H | $CF_3$ | H | C-F | Cl |
| 1 | Cl | H | $CF_3$ | H | C-F | Cl |
| 2 | Cl | H | $CF_3$ | H | C-F | Cl |
| 0 | Cl | H | $CF_3$ | F | C-Cl | Cl |
| 1 | Cl | H | $CF_3$ | F | C-Cl | Cl |
| 2 | Cl | H | $CF_3$ | F | C-Cl | Cl |
| 0 | Cl | H | $CF_3$ | Cl | C-Cl | Cl |
| 1 | Cl | H | $CF_3$ | Cl | C-Cl | Cl |
| 2 | Cl | H | $CF_3$ | Cl | C-Cl | Cl |
| 0 | Cl | H | $SO_2CF_3$ | H | CH | Cl |
| 1 | Cl | H | $SO_2CF_3$ | H | CH | Cl |
| 2 | Cl | H | $SO_2CF_3$ | H | CH | Cl |
| 0 | Cl | H | $SO_2CF_3$ | H | C-Cl | Cl |
| 1 | Cl | H | $SO_2CF_3$ | H | C-Cl | Cl |
| 2 | Cl | H | $SO_2CF_3$ | H | C-Cl | Cl |

Die in Tabelle 3 angegebenen Beispiele gelten im einzelnen jeweils für die ganz besonders bevorzugten Gruppen von Verbindungen der Formel (I), welche oben durch die Formeln (Ia), (Ib), (Ic), (Id) und (Ie) skizziert sind.

Die oben beschriebenen Ausgangsstoffe der Formel (I) für Verfahren (d) sind erfindungsgemäße, neue Verbindungen; sie können nach dem erfindungsgemäßen Verfahren (b) hergestellt werden.

Die beim erfindungsgemäßen Verfahren (d) weiter als Ausgangsstoffe zu verwendenden nucleophilen Verbindungen sind durch die Formel (IV) allgemein definiert.

In Formel (IV) haben n, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n, Y und Z angegeben wurden und M steht vorzugsweise für Wasserstoff, Natrium oder Kalium.

Beispiele für die Ausgangsstoffe der Formel (IV) sind in der nachstehenden Tabelle 4 aufgeführt.

Tabelle 4: Beispiele für die Ausgangsstoffe der Formel (IV)

$$M-(Y)_n-Z \quad (IV)$$

| M | n | Y | Z |
|---|---|---|---|
| H | 1 | O | $CH_3$ |
| Na | 1 | O | $CH_3$ |
| H | 1 | O | $C_2H_5$ |

Tabelle 4: - Fortsetzung

| M | n | Y | Z |
|---|---|---|---|
| Na | 1 | O | $C_2H_5$ |
| K | 1 | O | $C_2H_5$ |
| H | 1 | O | $C_3H_7$ |
| H | 1 | O | $CH(CH_3)_2$ |
| H | 1 | O | $C_4H_9$ |
| H | 1 | NH | H |
| H | 1 | NH | $CH_3$ |
| H | 1 | NH | $C_2H_5$ |
| H | 1 | NH | $C_3H_7$ |
| H | 1 | NH | $CH(CH_3)_2$ |
| H | 1 | NH | $C_4H_9$ |
| H | 1 | NH | $CH_2CH(CH_3)_2$ |
| H | 1 | NH | $CH_2COOC_2H_5$ |
| H | 1 | NH | $CH(CH_3)-COOC_2H_5$ |
| H | 1 | NH | $CH_2CH=CH_2$ |
| H | 1 | NH | 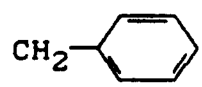 |
| H | 1 | NH | 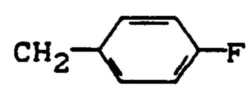 |
| H | 1 | NH | 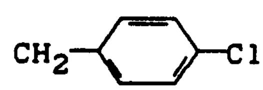 |
| H | 1 | NH | 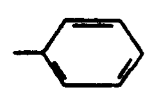 |
| H | 1 | NH | $CH_2CH_2OCH_3$ |

Tabelle 4: - Fortsetzung

| M | n | Y | Z |
|---|---|---|---|
| H | 1 | NH | 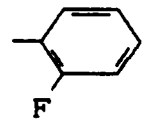 |
| H | 1 | NH | 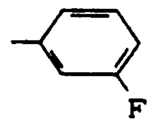 |
| H | 1 | NH | 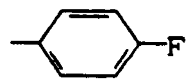 |
| H | 1 | NH | 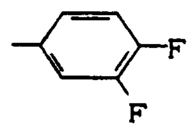 |
| H | 1 | NH | 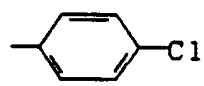 |
| H | 1 | NH | 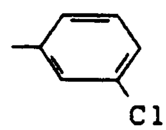 |
| H | 1 | NH | 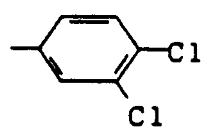 |
| H | 1 | NH | 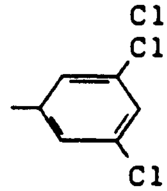 |
| H | 1 | NH | 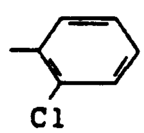 |
| H | 1 | NH | 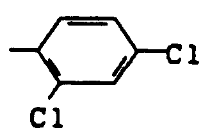 |

Tabelle 4: - Fortsetzung

| M | n | Y | Z |
|---|---|---|---|
| H | 1 | NH | 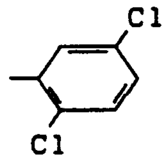 |
| H | 1 | NH | 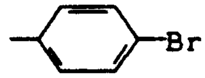 |
| H | 1 | NH | 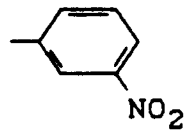 |
| H | 1 | NH | 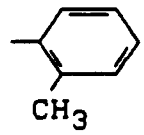 |
| H | 1 | NH | 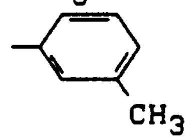 |
| H | 1 | NH | 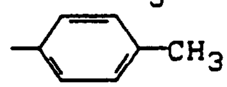 |
| H | 1 | NH | 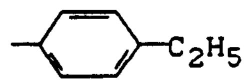 |
| H | 1 | NH | 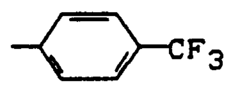 |
| H | 1 | NH | 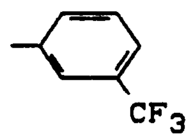 |
| H | 1 | NH | 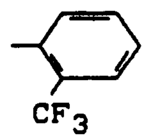 |
| H | 1 | NH | 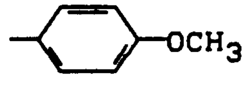 |

Tabelle 4: - Fortsetzung

| M | n | Y | Z |
|---|---|---|---|
| H | 1 | NH | 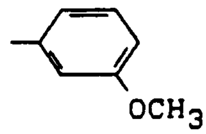 |
| H | 1 | NH | 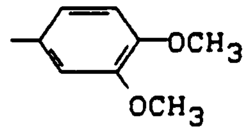 |
| H | 1 | NH | 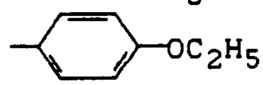 |
| H | 1 | NH | 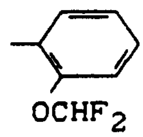 |
| H | 1 | NH | 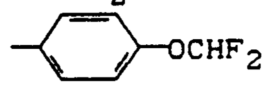 |
| H | 1 | NH | 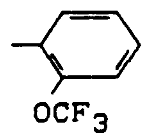 |
| H | 1 | NH | 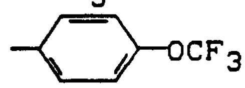 |
| H | 1 | NH | 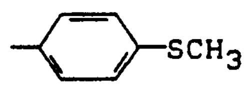 |
| H | 1 | NH | 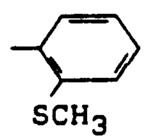 |
| H | 1 | NH | 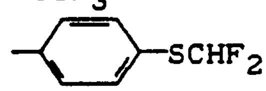 |
| H | 1 | NH | 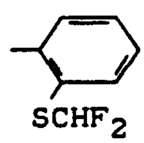 |

Tabelle 4: - Fortsetzung

| M | n | Y | Z |
|---|---|---|---|
| H | 1 | NH | 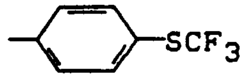 |
| H | 1 | NH | 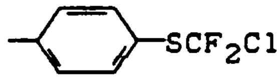 |
| H | 1 | NH | 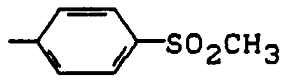 |
| H | 1 | NH | 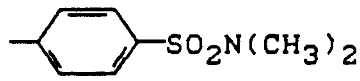 |
| H | 1 | NH | 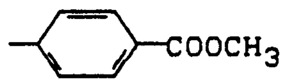 |
| H | 1 | NH | 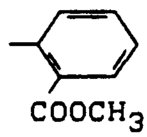 |
| H | 1 | NH | 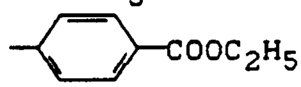 |
| H | 1 | NH | 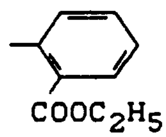 |
| H | 1 | NHCO | H |
| H | 1 | NHCO | $CH_3$ |
| H | 1 | NHCO | $C_2H_5$ |
| H | 1 | NHCO | $C_3H_7$ |
| H | 1 | NHCO | $C_4H_9$ |
| H | 1 | NHCO | 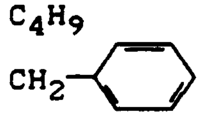 |

Tabelle 4: - Fortsetzung

| M | n | Y | Z |
|---|---|---|---|
| H | 1 | NHCO | 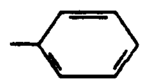 |
| H | 1 | NHCOO | $CH_3$ |
| H | 1 | NHCOO | $C_2H_5$ |
| H | 1 | NHCOO | $C_3H_7$ |
| H | 1 | NHCOO | $C_4H_9$ |
| H | 1 | NHCOO | $CH_2$ 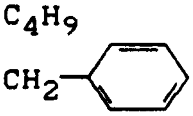 |
| H | 1 | NHCOO | 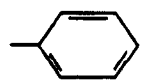 |
| H | 1 | $NHSO_2$ | $CH_3$ |
| H | 1 | $NHSO_2$ | $C_2H_5$ |
| H | 1 | $NHSO_2$ | $C_3H_7$ |
| H | 1 | $NHSO_2$ | $C_4H_9$ |
| H | 1 | $NHSO_2$ | $CH_2Cl$ |
| H | 1 | NHCO | $CH_2Cl$ |
| H | 1 | $NHSO_2$ | $CH_2CH_2Cl$ |
| H | 1 | NHCO | $CH_2CH_2Cl$ |
| H | 1 | NHCO | $CHCl_2$ |
| H | 1 | $NHSO_2$ | 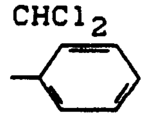 |
| H | 1 | $NHSO_2$ | 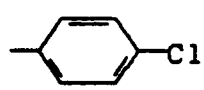 |

Tabelle 4: - Fortsetzung

| M | n | Y | Z |
|---|---|---|---|
| H | 1 | $NHSO_2$ | 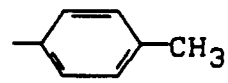 |
| H | 0 | - | 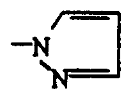 |
| K | 0 | - | 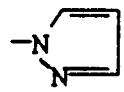 |
| H | 0 | - | 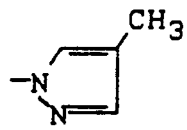 |
| H | 0 | - | 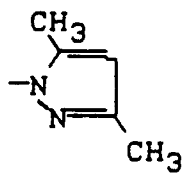 |
| H | 0 | - | 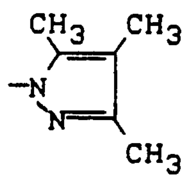 |
| H | 0 | - | 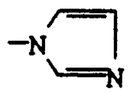 |
| H | 0 | - | 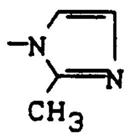 |
| H | 0 | - | 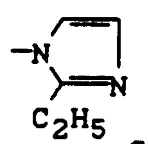 |
| H | 0 | - | 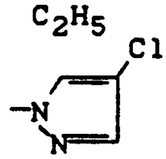 |

Die Ausgangsstoffe der Formel (IV) sind bekannte Synthesechemikalien.

Das erfindungsgemäße Verfahren (d) wird vorzugsweise unter Verwendung von Verdünnungsmitteln durchgeführt. Als Verdünnungsmittel kommen neben Wasser praktische alle inerten organischen Lösungsmitteln in Betracht, wie sie oben für das erfindungsgemäße Verfahren (a) angegeben sind.

Verfahren (d) wird gegebenenfalls in Gegenwart eines Säureakzeptors durchgeführt. Es kommen vor allem diejenigen Säurebindemittel in Betracht, die oben für Verfahren (a) angegeben sind, insbesondere jedoch die dort angegebenen aliphatischen, aromatischen oder heterocyclischen Amine.

Die Reaktionstemperaturen können bei dem erfindungsgemäßen Verfahren (d) in einem größeren Bereich variiert werden. Im allgemeinen arbeitet man bei Temperaturen zwischen -20 °C und +100 °C, vorzugsweise bei Temperaturen zwischen 0 °C und +80 °C.

Das erfindungsgemäße Verfahren (d) wird im allgemeinen unter Normaldruck durchgeführt. Es ist jedoch auch möglich, unter erhöhtem oder vermindertem Druck zu arbeiten.

Zur Durchführung des erfindungsgemäßen Verfahren (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (d) jeweils nach üblichen Methoden.

Die beim erfindungsgemäßen Verfahren (e) als Ausgangsstoffe zu verwendenden Verbindungen sind durch die Formel (I) mit der Maßgabe, daß Y für Sauerstoff, Schwefel oder NH steht und Z für Wasserstoff steht, allgemein definiert. In diesem Fall haben m, n, $R^1$, $R^2$, $R^3$, $R^4$ und X vorzugsweise bzw. insbesondere diejenigen Bedeutungen, die bereits oben im Rahmen der Beschreibung der erfindungsgemäßen Verbindungen der Formel (I) vorzugsweise bzw. als insbesondere bevorzugt angegeben wurden und Y steht vorzugsweise für NH.

Beispiele für die Ausgangsstoffe der Formel (I) für Verfahren (e) sind in der nachstehenden Tabelle 5 aufgeführt.

Tabelle 5: Beispiele für die Ausgangsstoffe zu Verfahren (e)

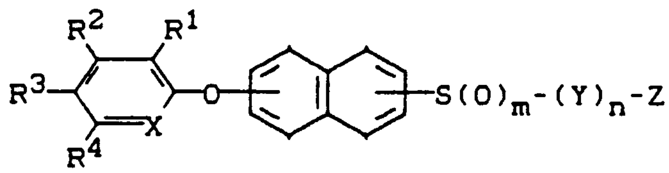

(I)

Hierbei gilt in jedem Einzelfall: Z = H

| m | n | $R^1$ | $R^2$ | $R^3$ | $R^4$ | X | Y |
|---|---|---|---|---|---|---|---|
| 0 | 0 | Cl | H | Cl | H | CH | - |
| 1 | 1 | Cl | H | Cl | H | CH | NH |
| 2 | 1 | Cl | H | Cl | H | CH | NH |
| 2 | 0 | Cl | H | Cl | H | CH | - |
| 0 | 0 | Cl | H | Cl | H | N | - |
| 1 | 1 | Cl | H | Cl | H | N | NH |
| 2 | 1 | Cl | H | Cl | H | N | NH |
| 2 | 0 | Cl | H | Cl | H | N | - |
| 0 | 0 | Cl | H | $CF_3$ | H | CH | - |
| 1 | 1 | Cl | H | $CF_3$ | H | CH | NH |
| 2 | 1 | Cl | H | $CF_3$ | H | CH | NH |
| 2 | 0 | Cl | H | $CF_3$ | H | CH | - |
| 0 | 0 | Cl | H | $CF_3$ | H | N | - |
| 1 | 1 | Cl | H | $CF_3$ | H | N | NH |
| 2 | 1 | Cl | H | $CF_3$ | H | N | NH |
| 2 | 0 | Cl | H | $CF_3$ | H | N | - |
| 0 | 0 | Cl | H | $CF_3$ | H | C-Cl | - |
| 1 | 1 | Cl | H | $CF_3$ | H | C-Cl | NH |
| 2 | 1 | Cl | H | $CF_3$ | H | C-Cl | NH |
| 2 | 0 | Cl | H | $CF_3$ | H | C-Cl | - |

Tabelle 5: - Fortsetzung

| m | n | $R^1$ | $R^2$ | $R^3$ | $R^4$ | X | Y |
|---|---|---|---|---|---|---|---|
| 0 | 0 | Cl | H | $CF_3$ | H | C-F | - |
| 1 | 1 | Cl | H | $CF_3$ | H | C-F | NH |
| 2 | 1 | Cl | H | $CF_3$ | H | C-F | NH |
| 2 | 0 | Cl | H | $CF_3$ | H | C-F | - |
| 0 | 0 | Cl | H | $CF_3$ | F | C-Cl | - |
| 1 | 1 | Cl | H | $CF_3$ | F | C-Cl | NH |
| 2 | 1 | Cl | H | $CF_3$ | F | C-Cl | NH |
| 2 | 0 | Cl | H | $CF_3$ | F | C-Cl | - |
| 0 | 0 | Cl | H | $CF_3$ | Cl | C-Cl | - |
| 1 | 1 | Cl | H | $CF_3$ | Cl | C-Cl | NH |
| 2 | 1 | Cl | H | $CF_3$ | Cl | C-Cl | NH |
| 2 | 0 | Cl | H | $CF_3$ | Cl | C-Cl | - |
| 2 | 1 | CN | H | $CF_3$ | H | CH | NH |
| 0 | 0 | Cl | H | $SO_2CF_3$ | H | CH | - |
| 1 | 1 | Cl | H | $SO_2CF_3$ | H | CH | NH |
| 2 | 1 | Cl | H | $SO_2CF_3$ | H | CH | NH |
| 2 | 0 | Cl | H | $SO_2CF_3$ | H | CH | - |
| 0 | 0 | Cl | H | $SO_2CF_3$ | H | C-Cl | - |
| 1 | 1 | Cl | H | $SO_2CF_3$ | H | C-Cl | NH |
| 2 | 1 | Cl | H | $SO_2CF_3$ | H | C-Cl | NH |
| 2 | 0 | Cl | H | $SO_2CF_3$ | H | C-Cl | - |

Die in Tabelle 5 angegebenen Beispiele gelten im einzelnen jeweils für die ganz besonders bevorzugten Gruppen von Verbindungen der Formel (I), welche oben durch die Formeln (Ia), (Ib), (Ic), (Id) und (Ie) skizziert sind.

Die oben beschriebenen Ausgangsstoffe der Formel (I) für Verfahren (e) sind erfindungsgemäße, neue Verbindungen; sie können nach den erfindungsgemäßen Verfahren (c) und (d) hergestellt werden.

Die beim erfindungsgemäßen Verfahren (e) weiter als Ausgangsstoffe zu verwendenden elektrophilen Verbindungen sind durch die Formel (V) allgemein definiert.

In Formel (V) haben n und $Z^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für n und Z angegeben wurden und $Z^2$ steht vorzugsweise für Chlor oder Brom; $Y^1$ steht für CO oder $SO_2$.

Beispiele für die Ausgangsstoffe der Formel (V) sind in der nachstehenden Tabelle 6 aufgeführt.

Tabelle 6: Beispiele für die Ausgangsstoffe der Formel (V)

$$Z^2-(Y^1)_n-Z^3 \quad (V)$$

| $Z^2$ | $Y^1$ | n | $Z^3$ |
|---|---|---|---|
| Br | - | 0 | $CH_3$ |
| Br | - | 0 | $C_2H_5$ |
| Br | - | 0 | $C_3H_7$ |
| Br | - | 0 | $CH(CH_3)_2$ |

Tabelle 6: - Fortsetzung

| $Z^2$ | $Y^1$ | n | $Z^3$ |
|---|---|---|---|
| Br | - | 0 | $C_4H_9$ |
| Br | - | 0 | $CH_2CH(CH_3)_2$ |
| Cl | - | 0 | $CHF_2$ |
| Br | - | 0 | $CH_2CH_2CH_2Cl$ |
| Br | - | 0 | $CH_2CH=CH_2$ |
| Cl | - | 0 | $CH_2COOCH_3$ |
| Br | - | 0 | $CH_2COOCH_3$ |
| Cl | - | 0 | $CH_2COOC_2H_5$ |
| Br | - | 0 | $CH_2COOC_2H_5$ |
| Cl | - | 0 | $CHCOOCH_3$<br>\|<br>$CH_3$ |
| Br | - | 0 | $CHCOOCH_3$<br>\|<br>$CH_3$ |
| Cl | - | 0 | $CHCOOC_2H_5$<br>\|<br>$CH_3$ |
| Br | - | 0 | $CHCOOC_2H_5$<br>\|<br>$CH_3$ |
| Br | - | 0 | $CH_2CH_2COOCH_3$ |
| Br | - | 0 | $CH_2CH_2COOC_2H_5$ |
| Cl | - | 0 | 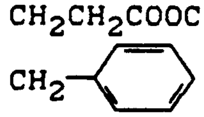 |
| Cl | - | 0 | 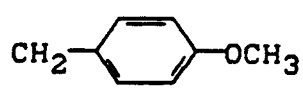 |

Tabelle 6: - Fortsetzung

| $Z^2$ | $Y^1$ | n | $Z^3$ |
|---|---|---|---|
| Cl | - | 0 | 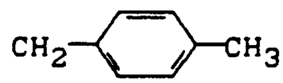 |
| Cl | CO | 1 | $CH_3$ |
| Cl | CO | 1 | $C_2H_5$ |
| Cl | CO | 1 | $C_3H_7$ |
| Cl | CO | 1 | $CH(CH_3)_2$ |
| Cl | CO | 1 | $CH_2Cl$ |
| Cl | CO | 1 | $CHCl_2$ |
| Cl | CO | 1 | $CCl_3$ |
| Cl | CO | 1 | 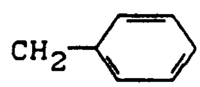 |
| Cl | CO | 1 | 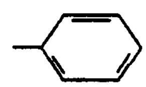 |
| Cl | CO | 1 | 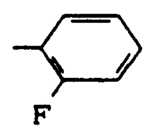 |
| Cl | CO | 1 | 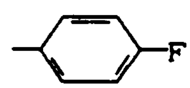 |
| Cl | CO | 1 | 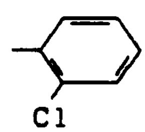 |
| Cl | CO | 1 | 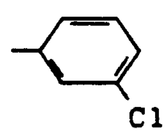 |
| Cl | CO | 1 | 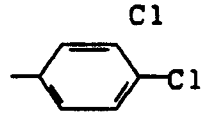 |

Tabelle 6: - Fortsetzung

| $Z^2$ | $Y^1$ | n | $Z^3$ |
|---|---|---|---|
| Cl | CO | 1 | 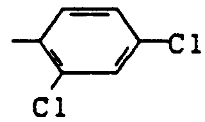 |
| Cl | CO | 1 | 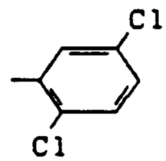 |
| Cl | CO | 1 | 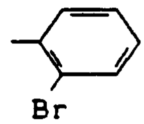 |
| Cl | CO | 1 | 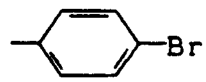 |
| Cl | CO | 1 | 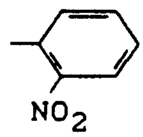 |
| Cl | CO | 1 | 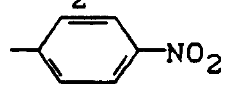 |
| Cl | CO | 1 | 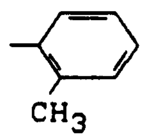 |
| Cl | CO | 1 | 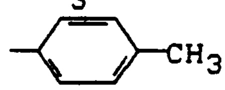 |
| Cl | $SO_2$ | 1 | $CH_3$ |
| Cl | $SO_2$ | 1 | $C_2H_5$ |
| Cl | $SO_2$ | 1 | $C_3H_7$ |
| Cl | $SO_2$ | 1 | $C_4H_9$ |
| Cl | $SO_2$ | 1 | $CH_2Cl$ |
| Cl | $SO_2$ | 1 | $CH_2CH_2Cl$ |

Tabelle 6: - Fortsetzung

| $Z^2$ | $Y^1$ | n | $Z^3$ |
|---|---|---|---|
| Cl | $SO_2$ | 1 | 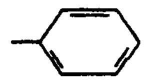 |
| Cl | $SO_2$ | 1 | 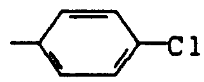 |
| Cl | $SO_2$ | 1 | 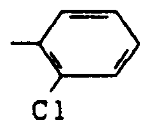 |
| Cl | $SO_2$ | 1 | 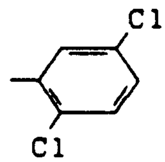 |
| Cl | $SO_2$ | 1 | 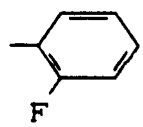 |
| Cl | $SO_2$ | 1 | 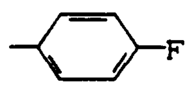 |
| Cl | $SO_2$ | 1 | 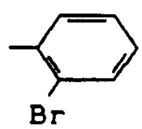 |
| Cl | $SO_2$ | 1 | 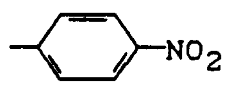 |
| Cl | $SO_2$ | 1 | 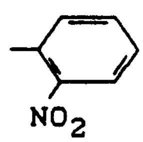 |
| Cl | $SO_2$ | 1 | 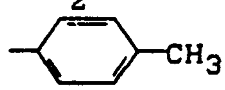 |
| Cl | $SO_2$ | 1 | 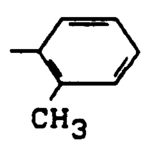 |

Tabelle 6: - Fortsetzung

| $Z^2$ | $Y^1$ | n | $Z^3$ |
|---|---|---|---|
| Cl | $SO_2$ | 1 | 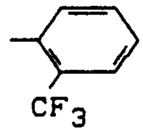 |
| Cl | $SO_2$ | 1 | 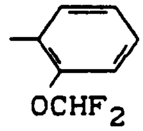 |
| Cl | $SO_2$ | 1 | 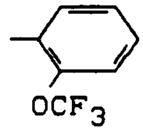 |
| Cl | $SO_2$ | 1 | 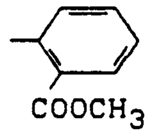 |

Die Ausgangsstoffe der Formel (V) sind bekannte organische Synthesechemikalien.

Das erfindungsgemäße Verfahren (e) wird vorzugsweise unter Verwendung von Verdünnungsmitteln durchgeführt. Als Verdünnungsmittel kommen neben Wasser praktisch alle inerten organischen Lösungsmittel in Betracht, wie sie oben für das erfindungsgemäße Verfahren (a) angegeben sind.

Verfahren (e) wird gegebenenfalls in Gegenwart eines Säureakzeptors durchgeführt. Es kommen vor allem diejenigen Säurebindemittel in Betracht, die oben für Verfahren (a) angegeben sind, insbesondere jedoch die dort angegebenen aliphatischen, aromatischen oder heterocyclischen Amine.

Die Reaktionstemperaturen können bei dem erfindungsgemäßen Verfahren (e) in einem größeren Bereich variiert werden. Im allgemeinen arbeitet man bei Temperaturen zwischen -20 °C und +100 °C, vorzugsweise bei Temperaturen zwischen 0 °C und +80 °C.

Das erfindungsgemäße Verfahren (e) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (e) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (e) jeweils nach üblichen Methoden.

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Wasser, Methanol, Ethanol oder Aceton und Zugabe einer geeigneten Base. Die Salze können dann - gegebenenfalls nach längerem Rühren - durch Einengen oder Absaugen isoliert werden.

In einzelnen Fällen (vgl. Herstellungsbeispiel 1) erhält man Salze von Verbindungen der Formel (I) unmittelbar bei der Durchführung des erfindungsgemäßen Herstellungsverfahrens.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich inbesondere zur selektiven Bekämpfung von dikotylen Unkräutern in monokotylen und dikotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischungen mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON), N,N-Dimethyl-N′-(3-chlor-4-methylphenyl)-harnstoff (CHLORTOLURON); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmittel ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

Herstellungsbeispiele

Beispiel 1

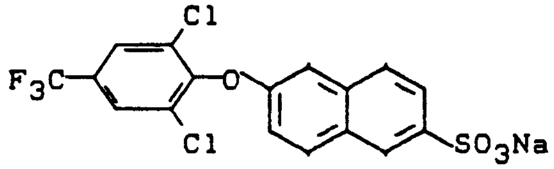

[Verfahren (a)]

124 g (0,5 Mol) 3,4,5-Trichlorbenzotrifluorid werden unter Rühren zu einer Mischung von 135 g (0,5 Mol) 6-Hydroxy-naphthalin-2-sulfonsäure-Natriumsalz, 30 g (0,54 Mol) Kaliumhydroxid-Pulver und 1000 ml Dimethylsulfoxid gegeben und das Reaktionsgemisch wird zunächst 3 Stunden bei 60 °C und dann 15 Stunden bei 20 °C gerührt. Nach Einengen wird der Rückstand mit Wasser digeriert und das kristallin angefallene Produkt durch Absaugen isoliert. Man erhält 209,5 g (91 % der Theorie) 6-(2,6-Dichlor-4-trifluormethyl-phenoxy)-naphthalin-2-sulfonsäure-Natriumsalz, welches oberhalb 310 °C unter Zersetzung schmilzt.

Beispiel 2

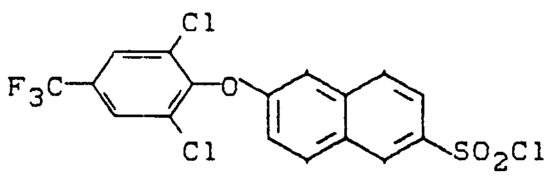

[Verfahren (b)]

Eine Mischung aus 2,3 g (5,0 mMol) 6-(2,6-Dichlor-4-trifluormethyl-phenoxy)-naphthalin-2-sulfonsäure-Natriumsalz, 1,6 g (7,5 mMol) Phosphor(V)-chlorid und 15 ml Phosphorylchlorid wird zunächst 6 Stunden bei 70 °C und dann 15 Stunden bei 20 °C gerührt, anschließend in Wasser gegossen und eine weitere Stunde gerührt. Das kristallin angefallene Produkt wird durch Absaugen isoliert.

Man erhält 1,8 g (79 % der Theorie) 6-(2,6-Dichlor-4-trifluormethyl-phenoxy)-naphthalin-2-sulfonsäurechlorid vom Schmelzpunkt 140 °C.

Beispiel 3

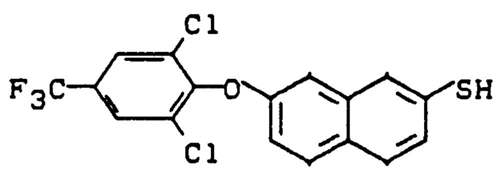

[Verfahren (c)]

Zu einer Mischung aus 12,5 g (0,19 Mol) Zinkstaub und 100 ml Dioxan werden nacheinander unter Rühren 12,5 g (0,025 Mol) 7-(2,6-Dichlor-4-trifluormethyl-phenoxy)-naphthalin-2-sulfonsäurechlorid und 25 ml konzentrierte Salzsäure gegeben. Das Reaktionsgemisch wird 5 Stunden under Rückfluß zum Sieden erhitzt und dann weitere 15 Stunden bei 20 °C gerührt. Nach Einengen der Rückstand in Methylenchlorid/Wasser aufgenommen, filtriert, das Filtrat geschüttelt, die organische Phase abgetrennt, mit Wasser gewaschen, mit Natriumsulfat getrocknet und filtriert.
Das filtrat wird eingeengt, der Rückstand mit Ethanol digeriert und das hierbei kristallin angefallene Produkt durch Absaugen isoliert.

Man erhält 4,8 g (49 % Theorie) 7-(2,6-Dichlor-4-trifluormethyl-phenoxy)-naphthalin-2-thiol vom Schmelzpunkt 98 °C.

Beispiel 4

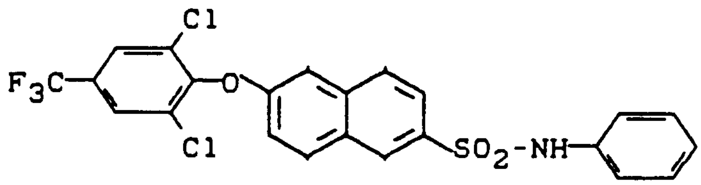

[Verfahren (d)]

Eine Mischung aus 4,6 g (0,01 Mol) 6-(2,6-Dichlor-4-trifluormethyl-phenoxy)-naphthalin-2-sulfonsäurechlorid, 0,9 g (0,01 Mol) Anilin, 1,1 g (0,011 Mol) Triethylamin und 100 ml Acetonitril wird 15 Stunden bei 20 °C gerührt. Das bei anschließendem Verdünnen mit Wasser kristallin angefallene Produkt wird durch Absaugen isoliert.

Man erhält 3,0 g (59 % der Theorie) 6-(2,6-Dichlor-4-trifluormethyl-phenoxy)-naphthalin-2-sulfonsäureanilid vom Schmelzpunkt 174 °C.

Beispiel 5

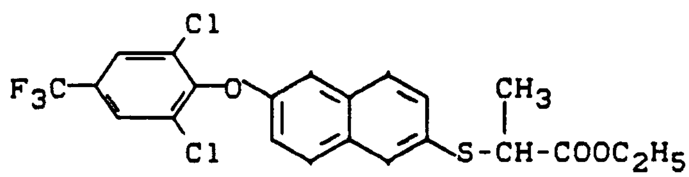

[Verfahren (e)]

Eine Mischung aus 1,9 g (5 mMol) 6-(2,6-Dichlor-4-trifluormethyl-phenoxy)-naphthalin-2-thiol, 1,0 g (5,5 mMol) α-Brom-propionsäureethylester, 0,7 g (5,5 mMol) Kaliumcarbonat und 50 ml Acetonitril wird 15 Stunden unter Rückfluß zum Sieden erhitzt und anschließend eingeengt. Der Rückstand wird in Methylenchlorid aufgenommen, zweimal mit Wasser gewaschen, mit Natriumsulfat getrocknet und filtriert. Vom Filtrat wird das Lösungsmittel bei 1 Torr (0,13 kPa) sorgfältig abdestilliert.

Man erhält 1,4 g (57 % der Theorie) α-[6-(2,6-Dichlor-4-trifluormethyl-phenoxy)-naphthalin-2-yl-thio]-propionsäureethylester als öligen Rückstand.

NMR-Daten:*)

$^1$H-NMR ($CDCl_3$, δ): 3,8 ppm

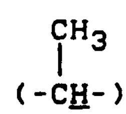

Beispiel 6

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.)

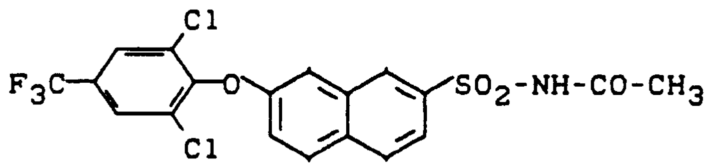

[Verfahren (e)]

Eine Mischung aus 2,1 g (5,0 mMol) 7-(2,6-Dichlor-4-trifluormethyl-phenoxy)-naphthalin-2-sulfonsäureamid, 0,4 g (5,5 mMol) Acetylchlorid, 1,1 g (5,5 mMol) Kaliumcarbonat und 50 ml Aceton wird 15 Stunden unter Rückfluß zum Sieden erhitzt. Nach Zugabe von Methylenchlorid wird mit Wasser gewaschen, mit Natriumsulfat getrocknet und filtriert. Vom Filtrat wird das Lösungsmittel abdestilliert, der Rückstand mit Ethanol digeriert und das hierbei kristallin angefallene Produkt durch Absaugen isoliert.
Man erhält 1,1 g (46 % der Theorie) N-Acetyl-7-(2,6-dichlor-4-trifluormethyl-phenoxy)-naphthalin-2-sulfonsäureamid vom Schmelzpunkt 137 °C.

Analog zu den Beispielen 1 bis 6 sowie entsprechend der allgemeinen Beschreibung der erfindungsgemäßen Herstellungsverfahren können die in der nachstehenden Tabelle 7 aufgeführten Verbindungen der Formel (I) - genauer spezifiziert durch die "Isomerengruppen" der Formeln (Ia), (Ib), (Ic), (Id) und (Ie) - hergestellt werden.

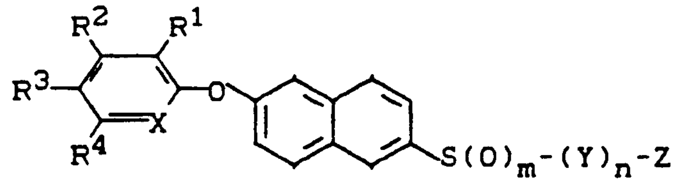

(Ia)

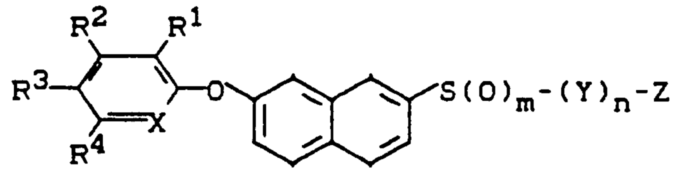

(Ib)

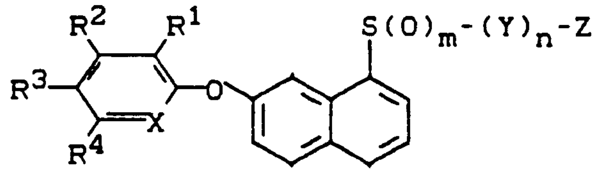

(Ic)

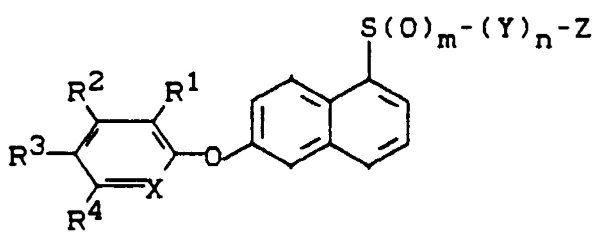

(Id)

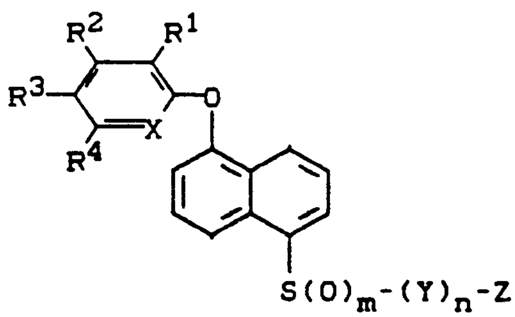

(Ie)

<u>Tabelle 7</u>: - Beispiele für die Verbindungen der Formel (I)

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 7 | Ia | 0 | 0 | Cl | H | $CF_3$ | H | C-Cl | - | H | Fp:129° C |
| 8 | Ib | 2 | 1 | Cl | H | $CF_3$ | H | C-Cl | O | Na | Fp:262° C |
| 9 | Ib | 2 | 0 | Cl | H | $CF_3$ | H | C-Cl | - | Cl | Fp:176° C |
| 10 | Ia | 2 | 1 | Cl | H | $CF_3$ | H | C-Cl | NH | H | Fp:204° C |
| 11 | Ib | 2 | 1 | Cl | H | $CF_3$ | H | C-Cl | O | $C_2H_5$ | Fp:105° C |
| 12 | Ib | 2 | 1 | Cl | H | $CF_3$ | H | C-Cl | NH | 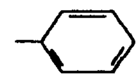 | Fp:150° C |
| 13 | Ib | 2 | 1 | Cl | H | $CF_3$ | H | C-Cl | NH | $C_2H_5$ | Fp:161° C |
| 14 | Ia | 2 | 1 | Cl | H | $CF_3$ | H | C-Cl | NH | $C_2H_5$ | Fp:135° C |
| 15 | Ib | 2 | 1 | Cl | H | $CF_3$ | H | C-Cl | NH | H | Fp:208° C |
| 16 | Ia | 2 | 0 | Cl | H | $CF_3$ | H | C-Cl | - | 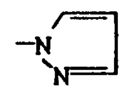 | Fp:171° C |
| 17 | Ia | 2 | 0 | Cl | H | $CF_3$ | H | C-Cl | - | 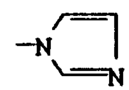 | Fp:172° C |

<u>Tabelle 7</u>: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 18 | Ia | 2 | 0 | Cl | H | $CF_3$ | H | C-Cl | - | 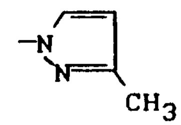 | Fp:168° C |
| 19 | Ia | 2 | 1 | Cl | H | $CF_3$ | H | C-Cl | O | H | Fp:280° C |
| 20 | Ib | 0 | 0 | Cl | H | $CF_3$ | H | C-Cl | - | $CH_3$ | Fp:120° C |
| 21 | Ia | 0 | 0 | Cl | H | $CF_3$ | H | C-Cl | - | $CH_3$ | Fp:123° C |
| 22 | Ib | 2 | 1 | Cl | H | $CF_3$ | H | C-Cl | O | H | Fp:148° C |
| 23 | Ia | 0 | 0 | Cl | H | $CF_3$ | H | C-Cl | - | $-CHCOOC_2H_5$<br>$CH_3$ | $^1H$-NMR*)<br>3,9ppm<br>$-C\underline{H}-COOC_2H_5$<br>$CH_3$ |
| 24 | Ib | 0 | 0 | Cl | H | $CF_3$ | H | C-Cl | - | $-CHCOOCH_3$<br>$CH_3$ | $^1H$-NMR*)<br>3,9ppm<br>$-C\underline{H}-COOCH_3$<br>$CH_3$ |
| 25 | Ib | 2 | 1 | Cl | H | $CF_3$ | H | C-F | O | Na | Fp:207° C |
| 26 | Ib | 0 | 0 | Cl | H | $CF_3$ | H | C-Cl | - | $-CH_2CH_2COOC_2H_5$ | $^1H$-NMR*)<br>3,2; 2,6ppm<br>$-C\underline{H}_2-C\underline{H}_2-$ |

Tabelle 7: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 27 | Ib | 2 | 0 | Cl | H | $CF_3$ | H | C-F | - | Cl | Fp:165° C |
| 28 | Ib | 2 | 1 | Cl | H | $CF_3$ | H | C-F | NH | H | Fp:188° C |
| 29 | Ib | 0 | 0 | Cl | H | $CF_3$ | H | C-Cl | - | (R)-$\overset{*}{C}HCOOCH_3$<br>│<br>$CH_3$ | $^1H$-NMR*)<br>3,9ppm<br>-C<u>H</u>-$COOCH_3$<br>│<br>$CH_3$ |
| 30 | Ib | 0 | 0 | Cl | H | $CF_3$ | H | C-F | - | H | Fp:89° C |
| 31 | Ib | 0 | 0 | Cl | H | $CF_3$ | H | C-F | - | -CH-$COOCH_3$<br>│<br>$CH_3$ | $^1H$-NMR*)<br>3,9ppm<br>-C<u>H</u>-$COOCH_3$<br>│<br>$CH_3$ |
| 32 | Ib | 0 | 0 | Cl | H | $CF_3$ | H | C-F | - | $-CH_2CH_2COOC_2H_5$ | $^1H$-NMR*)<br>3,2; 2,6ppm<br>$-CH_2-CH_2-$ |
| 33 | Ib | 0 | 0 | Cl | H | $CF_3$ | H | C-F | - | (R)-$\overset{*}{C}HCOOCH_3$<br>│<br>$CH_3$ | Fp:55° C |
| 34 | Ib | 2 | 0 | Cl | H | $CF_3$ | H | C-Cl | - | 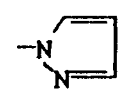 | Fp:209° C |

Tabelle 7: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 35 | Ia | 2 | 1 | Cl | H | $CF_3$ | H | C-Cl | -NHCO- | $C_2H_5$ | Fp:162° C |
| 36 | Ia | 2 | 1 | Cl | H | $CF_3$ | H | C-Cl | -NHCO- | $C_4H_9$ | Fp:163° C |
| 37 | Ia | 2 | 1 | Cl | H | $CF_3$ | H | C-Cl | -NHCO- | $CH_3$ | Fp:206° C |
| 38 | Id | 2 | 1 | Cl | H | $CF_3$ | H | C-Cl | O | Na | Fp:>260° C |
| 39 | Ie | 2 | 1 | Cl | H | $CF_3$ | H | C-Cl | O | Na | Fp:>260° C |
| 40 | Id | 2 | 0 | Cl | H | $CF_3$ | H | C-Cl | - | Cl | Fp:145° C |
| 41 | Ie | 2 | 0 | Cl | H | $CF_3$ | H | C-Cl | - | Cl | Fp:135° C |
| 42 | Id | 2 | 1 | Cl | H | $CF_3$ | H | C-Cl | O | H | Fp:105° C |
| 43 | Ie | 2 | 1 | Cl | H | $CF_3$ | H | C-Cl | O | H | Fp:110° C |
| 44 | Ie | 0 | 0 | Cl | H | $CF_3$ | H | C-Cl | - | H | Fp:185° C |
| 45 | Ib | 2 | 1 | Cl | H | $CF_3$ | H | C-Cl | NH | $-\underset{CH_3}{\underset{\vert}{CH}}-COOC_2H_5$ | Fp:128° C |
| 46 | Ib | 2 | 1 | Cl | H | $CF_3$ | H | C-F | NH | $-\underset{CH_3}{\underset{\vert}{CH}}-COOC_2H_5$ | Fp:97° C |

Tabelle 7: - Beispiele für die Verbindungen der Formel (I) / Fortsetzung

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 47 | Ie | 0 | 0 | Cl | H | $CF_3$ | H | C-Cl | - | $-CH-COOC_2H_5$<br>\|<br>$CH_3$ | Fp:98° C |
| 48 | Ie | 0 | 0 | Cl | H | $CF_3$ | H | C-Cl | - | $-CH-COOCH_3$<br>\|<br>$CH_3$ | (amorph) |
| 49 | Ic | 0 | 0 | Cl | H | $CF_3$ | H | C-Cl | - | H | Fp:201° C |
| 50 | Id | 2 | 1 | Cl | H | $CF_3$ | H | C-Cl | NH | H | Fp:185° C |
| 51 | Ic | 0 | 0 | Cl | H | $CF_3$ | H | C-Cl | - | $-CH-COOC_2H_5$<br>\|<br>$CH_3$ | (amorph) |
| 52 | Ic | 0 | 0 | Cl | H | $CF_3$ | H | C-Cl | - | $-CH-COOCH_3$<br>\|<br>$CH_3$ | Fp:68° C |
| 53 | Ic | 2 | 1 | Cl | H | $CF_3$ | H | C-Cl | O | H | (amorph) |
| 54 | Ie | 2 | 1 | Cl | H | $CF_3$ | H | C-Cl | -NHCOO- | $-C_2H_5$ | Fp:133° C |
| 55 | Id | 2 | 1 | Cl | H | $CF_3$ | H | C-F | O | Na | Fp:>260° C |
| 56 | Id | 2 | 1 | Cl | H | $CF_3$ | H | C-Cl | O | Na | Fp:>260° C |

Tabelle 7: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 57 | Ic | 2 | 1 | Cl | H | $CF_3$ | H | C-Cl | NH | H | Fp:171° C |
| 58 | Ie | 2 | 0 | Cl | H | $CF_3$ | H | C-Cl | - | 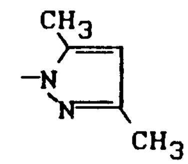 | Fp:158° C |
| 59 | Ic | 2 | 0 | Cl | H | $CF_3$ | H | C-Cl | - | 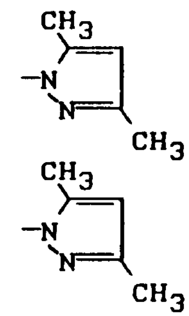 | Fp:113° C |
| 60 | Id | 2 | 0 | Cl | H | $CF_3$ | H | C-Cl | - | | Fp:167° C |
| 61 | Ie | 2 | 0 | Cl | H | $CF_3$ | H | C-Cl | - | 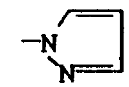 | Fp:84° C |
| 62 | Ic | 2 | J | Cl | H | $CF_3$ | H | C-Cl | - | 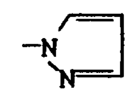 | Fp:163° C |
| 63 | Id | 2 | 0 | Cl | H | $CF_3$ | H | C-F | - | Cl | Fp:117° C |
| 64 | Ie | 2 | 0 | Cl | H | $CF_3$ | H | C-F | - | Cl | Fp:89° C |

Tabelle 7: - Beispiele für die Verbindungen der Formel (I) / Fortsetzung

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 65 | Ib | 0 | 0 | Cl | H | $CF_3$ | H | C-Cl | - | $CH_3$<br>$-CCOOC_2H_5$<br>$CH_3$ | |
| 66 | Ib | 0 | 0 | Cl | H | $CF_3$ | H | C-Cl | - | $-CHCOOC_2H_5$<br>$C_2H_5$ | |
| 67 | Ib | 0 | 0 | Cl | H | $CF_3$ | H | C-Cl | - | $-CHCOOCHCOOC_2H_5$<br>$CH_3$ $CH_3$ | |
| 68 | Ib | 0 | 0 | Cl | H | Cl | H | N | - | $-CHCOOCH_3$<br>$CH_3$ | |
| 69 | Ib | 0 | 0 | CN | H | $CF_3$ | H | CH | - | $-CHCOOCH_3$<br>$CH_3$ | |
| 70 | Ib | 0 | 0 | CN | H | $CF_3$ | H | CH | - | H | |
| 71 | Ib | 2 | 0 | CN | H | $CF_3$ | H | CH | - | Cl | Fp. 163° C |
| 72 | Ib | 0 | 0 | Cl | H | Cl | H | N | - | H | |
| 73 | Ib | 2 | 0 | Cl | H | Cl | H | N | - | Cl | Fp. 140° C |

Tabelle 7: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 74 | Ib | 0 | 0 | Cl | H | $CF_3$ | Cl | C-Cl | - | $-CH-COOCH_3$<br>\|<br>$CH_3$ | |
| 75 | Ib | 0 | 0 | Cl | H | $CF_3$ | Cl | C-Cl | - | H | |
| 76 | Ib | 2 | 0 | Cl | H | $CF_3$ | Cl | C-Cl | - | Cl | |
| 77 | Ib | 2 | 0 | Cl | H | $CF_3$ | F | C-Cl | - | Cl | |
| 78 | Ib | 0 | 0 | Cl | H | $CF_3$ | F | C-Cl | - | H | |
| 79 | Ib | 0 | 0 | Cl | H | $CF_3$ | H | C-Cl | - | $-CH_2COOCH_3$ | Fp:77° C |
| 80 | Ib | 2 | 1 | Cl | H | $CF_3$ | H | C-F | -NHCO- | $CH_3$ | Fp:102° C |
| 81 | Ib | 2 | 1 | Cl | H | $CF_3$ | H | C-Cl | $-NHSO_2-$ | 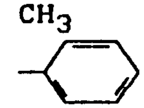 | Fp:275° C |
| 82 | Ib | 2 | 0 | Cl | H | $CF_3$ | H | CH | - | Cl | |
| 83 | Ic | 2 | 1 | Cl | H | $CF_3$ | H | C-Cl | O | Na | (amorph) |
| 84 | Ic | 2 | 0 | Cl | H | $CF_3$ | H | C-Cl | - | Cl | Fp:118° C |
| 85 | Ie | 2 | 1 | Cl | H | $CF_3$ | H | C-Cl | NH | H | Fp:210° C |
| 86 | Ib | 2 | 1 | Cl | H | $CF_3$ | H | C-Cl | NH | $-CH_2COOC_2H_5$ | Fp:131° C |

Tabelle 7: - Beispiele für die Verbindungen der Formel (I) / Fortsetzung

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 87 | Ib | 2 | 1 | Cl | H | $CF_3$ | H | C-F | NH | $-CH_2COOC_2H_5$ | Fp: 88° C |
| 88 | Id | 0 | 0 | Cl | H | $CF_3$ | H | C-Cl | - | H | (amorph) |
| 89 | Id | 0 | 0 | Cl | H | $CF_3$ | H | C-Cl | - | $-CHCOOC_2H_5$<br>\|<br>$CH_3$ | (amorph) |
| 90 | Id | 0 | 0 | Cl | H | $CF_3$ | H | C-Cl | - | $-CHCOOCH_3$<br>\|<br>$CH_3$ | (amorph) |
| 91 | Ie | 2 | 1 | Cl | H | $CF_3$ | H | C-Cl | -NHCO- | $CH_3$ | Fp: 142° C |
| 92 | Id | 2 | 0 | Cl | H | $CF_3$ | H | C-F | - | 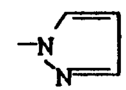 | Fp: 151° C |
| 93 | Ie | 2 | 0 | Cl | H | $CF_3$ | H | C-F | - | 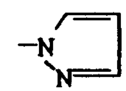 | (amorph) |
| 94 | Ib | 0 | 0 | Cl | H | $CF_3$ | H | C-F | - | $-CH_2COOCH_3$ | $^1H$-NMR*) 3,7 ppm $-C\underline{H}_2-$ |
| 95 | Ia | 2 | 1 | Cl | H | $CF_3$ | H | Cl | -NHCO- | $-C_2H_5$ | Fp: 270° C |

<u>Tabelle 7</u>: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 96 | Ic | 2 | 1 | Cl | H | $CF_3$ | H | C-Cl | -NHCOO- | $-C_2H_5$ | Fp:67° C |
| 97 | Ie | 2 | 1 | Cl | H | $CF_3$ | H | C-F | -NH- | $-(CH_3)_2OCH_3$ | Fp:103° C |
| 98 | Id | 0 | 0 | F | H | $CF_3$ | H | C-Cl | - | H | |
| 99 | Ie | 0 | 0 | F | H | $CF_3$ | H | C-Cl | - | H | Fp:74° C |
| 100 | Ib | 0 | 0 | Cl | H | $CF_3$ | H | C-F | - | $-CH_2COOC_2H_5$ | |
| 101 | Ie | 0 | 0 | F | H | $CF_3$ | H | C-Cl | - | $-CHCOOC_2H_5$<br>$CH_3$ | |
| 102 | Ie | 0 | 0 | F | H | $CF_3$ | H | C-Cl | - | $-CH-COOC_2H_5$<br>$CH_3$ | |
| 103 | Id | 0 | 0 | F | H | $CF_3$ | H | C-Cl | - | $-CH-COOCH_3$<br>$CH_3$ | |
| 104 | Id | 0 | 0 | Cl | H | $CF_3$ | H | C-F | - | $-CH-COOC_2H_5$<br>$CH_3$ | |
| 105 | Id | 0 | 0 | Cl | H | $CF_3$ | H | C-F | - | $-CH_2COOC_2H_5$ | |

Tabelle 7: - Beispiele für die Verbindungen der Formel (I) / Fortsetzung

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 106 | Ib | 2 | 1 | Cl | H | $CF_3$ | H | C-F | O | H | Öl |
| 107 | Ib | 0 | 0 | Cl | H | $CF_3$ | H | C-Cl | - | $-C(COOC_2H_5)_2$<br>$CH_3$ | Öl |
| 108 | Id | 2 | 0 | Cl | H | Cl | H | N | - | Cl | Fp. 107° C |
| 109 | Id | 2 | 1 | Cl | H | Cl | H | N | O | Na | Fp. >260° C |
| 110 | Ic | 0 | 0 | F | H | $CF_3$ | H | C-Cl | - | $-CH-COOC_2H_5$<br>$CH_3$ | Öl |
| 111 | Ic | 0 | 0 | F | H | $CF_3$ | H | C-Cl | - | $-CH-COOCH_3$<br>$CH_3$ | Öl |
| 112 | Ic | 0 | 0 | F | H | $CF_3$ | H | C-Cl | - | $-CH_2-COOC_2H_5$ | Öl |
| 113 | Id | 2 | 1 | Cl | H | $CF_3$ | H | C-Cl | -N-COO-<br>H | $-C_2H_5$ | Fp. 155° C |
| 114 | Ie | 2 | 1 | Cl | H | $CF_3$ | H | C-Cl | -N-<br>H | $-CH_2COOC_2H_5$ | Fp. 115° C |

Tabelle 7: - Beispiele für die Verbindungen der Formel (I) / Fortsetzung

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 115 | Ib | 2 | 1 | Cl | H | $CF_3$ | H | N | O | Na | Fp. > 300° C |
| 116 | Ib | 2 | 0 | Cl | H | $CF_3$ | H | N | - | Cl | Fp. 147° C |
| 117 | Ib | 2 | 0 | Cl | H | $CF_3$ | H | C-Cl | - | $-CHCOOCH_3$<br>$CH_3$ | Fp. 122° C |
| 118 | Ib | 2 | 1 | CN | H | $CF_3$ | H | CH | O | Na | Fp. > 300° C |
| 119 | Ib | 2 | 1 | Cl | H | $CF_3$ | H | N | NH | H | Fp. 192° C |
| 120 | Ib | 0 | 0 | Cl | H | $CF_3$ | H | N | - | $-CHCOOCH_3$<br>$CH_3$ | (amorph) |
| 121 | Ib | 2 | 1 | CN | H | $CF_3$ | H | CH | NH | H | Fp. 158° C |
| 122 | Id | 0 | 0 | CN | H | $CF_3$ | H | CH | - | H | Fp. 100° C |
| 123 | Ib | 2 | 1 | Cl | H | $CF_3$ | H | N | -NHCO- | $-CH_3$ | Fp. 172° C |
| 124 | Ib | 2 | 1 | CN | H | $CF_3$ | H | CH | -NHCO- | $-CH_3$ | Fp. 205° C |

Tabelle 7: - Beispiele für die Verbindungen der Formel (I) / Fortsetzung

| Bsp. Nr. | Iso-meren-Gruppe | m | n | $R^1$ | $R^2$ | $R^3$ | $R^4$ | X | Y | Z | physikal. Daten |
|---|---|---|---|---|---|---|---|---|---|---|---|
| 125 | Ib | 0 | 0 | CN | H | $CF_3$ | H | CH | - | $-CH_2COOCH_3$ | (amorph) |
| 126 | Ib | 0 | 0 | Cl | H | $CF_3$ | H | C-F | - | $-C(CH_3)_2COOC_2H_5$ | (amorph) |
| 127 | Ia | 0 | 0 | Cl | H | $CF_3$ | H | C-Cl | - | $-CH_2COOCH_3$ | Fp. 114° C |
| 128 | Ib | 0 | 0 | Cl | H | $CF_3$ | H | C-Cl | - | $-CHCOOH$<br>\|<br>$CH_3$ | Fp. 148° C |
| 129 | Ib | 2 | 1 | Cl | H | Cl | H | N | O | Na | Fp. > 260° C |
| 130 | Ib | 0 | 0 | Cl | H | Cl | H | N | - | $-CH_2COOCH_3$ | Fp. 115° C |
| 131 | Id | 2 | 1 | Cl | H | $CF_3$ | H | C-Cl | NH | $-CHCOOC_2H_5$<br>\|<br>$CH_3$ | Fp. 103° C |
| 132 | Ie | 2 | 1 | Cl | H | $CF_3$ | H | C-F | O | Na | Fp. > 260° C |
| 133 | Ic | 2 | 0 | Cl | H | $CF_3$ | H | C-F | - | 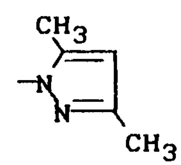 | Fp. 78° C |

Tabelle 7: - Beispiele für die Verbindungen der Formel (I) / Fortsetzung

| Bsp. Nr. | Isomeren-Gruppe | m | n | $R^1$ | $R^2$ | $R^3$ | $R^4$ | X | Y | Z | physikal. Daten |
|---|---|---|---|---|---|---|---|---|---|---|---|
| 134 | Id | 2 | 1 | Cl | H | Cl | H | N | NH | H | Fp. 103° C |
| 135 | Id | 2 | 1 | Cl | H | $CF_3$ | H | N | O | Na | Fp. >250° C |
| 136 | Id | 2 | 0 | Cl | H | $CF_3$ | H | N | - | Cl | Fp. 138° C |
| 137 | Id | 2 | 1 | Cl | H | $CF_3$ | H | N | NH | H | Fp. 140° C |
| 138 | Id | 2 | 1 | Cl | H | $CF_3$ | H | N | -NH-COO- | $C_2H_5$ | Fp. 88° C |
| 139 | Id | 0 | 0 | Cl | H | $CF_3$ | H | C-F | - | $-(CH_2)_3COOC_2H_5$ | (amorph) |
| 140 | Id | 0 | 0 | Cl | H | $CF_3$ | H | C-F | - | $-(CH_2)_2COOC_2H_5$ | (amorph) |
| 141 | Ic | 0 | 0 | Cl | H | $CF_3$ | H | C-Cl | - | $-CH_2COOCH_3$ | (amorph) |
| 142 | Ic | 0 | 0 | Cl | H | $CF_3$ | H | C-F | - | $-CH_2COOCH_3$ | (amorph) |

*) Die $^1$H-NMR-Spektren wurden aufgenommen in $DCCl_3$ mit Tetramethylsilan als innerem Standard. Angegeben sind die chemischen Verschiebungen als δ-Werte für die Gruppierungen $-\underline{CH_2}-$ bzw. $-CH_2\underline{CH_2}-$ bzw. $-\underline{CH}-CH_3$.

Anwendungsbeispiel

Im folgenden Anwendungsbeispiel wird die nachstehend aufgeführte Verbindung als Vergleichssubstanz herangezogen:

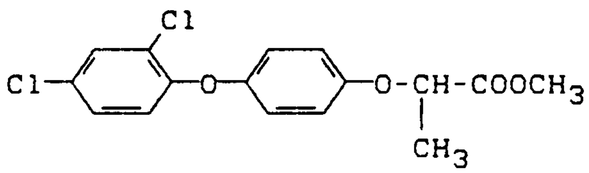

(A)

α-[4-(2,4-Dichlor-phenoxy)-phenoxy]-propionsäure-methylester (Diclofop-methyl) (bekannt aus DE-OS 22 23 894/Beispiel 86).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit gegenüber dem Stand der Technik zeigen in diesem Test z.B. die Verbindungen gemäß den Herstellungsbeispielen: (9), (23), (24), (94) und (142).

**Patentansprüche**

**1.** (Hetero)Aryloxynaphthaline mit über Schwefel gebundenen Substituenten der allgemeinen Formel (I)

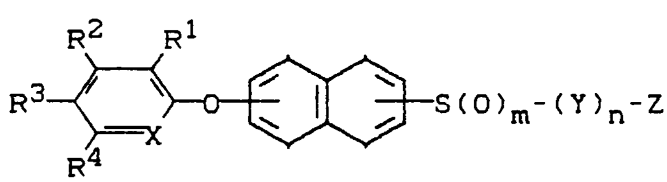

(I)

in welcher

- m für die Zahlen 0, 1 oder 2 steht,
- n für die Zahlen 0 oder 1 steht,
- $R^1$ für Wasserstoff, Halogen, Cyano oder Trifluormethyl steht,
- $R^2$ für Wasserstoff oder Halogen steht,
- $R^3$ für Halogen, Trifluormethyl, Trifluormethoxy, Trifluormethylthio oder Trifluormethylsulfonyl steht,
- $R^4$ für Wasserstoff oder Halogen steht,
- X für Stickstoff oder die Gruppierung C-$R^5$ steht, worin
- $R^5$ für Wasserstoff oder Halogen steht,
- Y für Sauerstoff oder eine der Gruppierungen

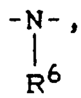, 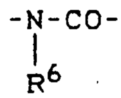, 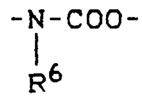, 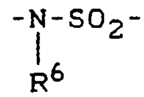

steht,
worin

$R^6$ für Wasserstoff, Alkyl, -CO-Z oder $-SO_2-Z$ steht und

Z für Wasserstoff, Halogen oder einen gegebenenfalls substituierten Rest aus der Reihe Alkyl, Cycloalkyl, Cycloalkylalkyl, Alkenyl, Alkinyl, Aralkyl, Aryl und Heteroaryl steht,

sowie Salze von Verbindungen der Formel (I).

**2.** Verbindungen der allgemeinen Formel (I) gemäß Anspruch 1, dadurch gekennzeichnet, daß darin

m für die Zahlen 0, 1 oder 2 steht,

n für die Zahlen 0 oder 1 steht,

$R^1$ für Wasserstoff, Fluor, Chlor, Brom, Cyano oder Trifluormethyl steht,

$R^2$ für Wasserstoff, Fluor oder Chlor steht,

$R^3$ für Fluor, Chlor, Brom, Trifluormethyl, Trifluormethoxy, Trifluormethylthio oder Trifluormethylsulfonyl steht,

$R^4$ für Wasserstoff, Fluor oder Chlor steht,

X für Stickstoff oder die Gruppierung $C-R^5$ steht, worin

$R^5$ für Wasserstoff, Fluor, Chlor oder Brom steht,

Y für Sauerstoff oder eine der Gruppierungen

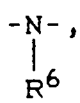, 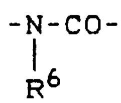, 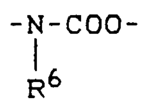, 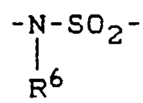

steht,
worin

$R^6$ für Wasserstoff, $C_1$-$C_4$-Alkyl, -CO-Z oder $-SO_2-Z$ steht und

Z für Wasserstoff, Halogen, für gegebenenfalls durch Halogen, Cyano, Carboxy, $C_1$-$C_4$-Alkoxy, $C_1$-$C_4$-Alkylcarbonyl, $C_1$-$C_4$-Alkoxy-carbonyl, $C_1$-$C_4$-Alkoxy-carbonyl-$C_1$-$C_4$-alkoxy-carbonyl und/oder $C_1$-$C_4$-Alkylamino-carbonyl substituiertes $C_1$-$C_6$-Alkyl, für jeweils gegebenenfalls durch Halogen und/oder $C_1$-$C_4$-Alkyl substituiertes $C_3$-$C_6$-Cycloalkyl oder $C_3$-$C_6$-Cycloalkyl-$C_1$-$C_4$-alkyl, für jeweils gegebenenfalls durch Halogen, Cyano, Carboxy und/oder $C_1$-$C_4$-Alkoxy-carbonyl substituiertes $C_2$-$C_6$-Alkenyl oder $C_2$-$C_6$-Alkinyl, für jeweils gegebenenfalls durch Halogen, Cyano, Carboxy, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfonyl, $C_1$-$C_4$-Alkylsulfonyl, Di-($C_1$-$C_2$-alkyl)-aminosulfonyl, Di-($C_1$-$C_2$-alkyl)-aminocarbonyl, Di-($C_1$-$C_2$-alkyl)-amino, $C_1$-$C_4$-Alkoxy-carbonyl und/oder $C_1$-$C_2$-Alkylendioxy substituiertes Phenyl, Naphthyl, Phenyl-$C_1$-$C_4$-alkyl oder Naphthyl-$C_1$-$C_4$-alkyl steht, oder für einen gegebenenfalls durch Halogen, $C_1$-$C_4$-Alkyl, $C_1$-$C_2$-Halogenalkyl, $C_1$-$C_4$-Alkoxy und/oder $C_1$-$C_2$-Halogenalkoxy substituierten Heterocyclus aus der Reihe Pyrazolyl, Imidazolyl, Triazolyl steht.

**3.** Verbindungen der allgemeinen Formel (I) gemäß Anspruch 1, dadurch gekennzeichnet, daß darin

$R^1$ für Cyano, Fluor oder Chlor steht,

$R^2$ für Wasserstoff, Fluor oder Chlor steht,

$R^3$ für Chlor, Trifluormethyl oder Trifluormethylsulfonyl steht,

$R^4$ für Wasserstoff, Fluor oder Chlor steht,

X für Stickstoff oder die Gruppierung $C-R^5$ steht, worin

$R^5$ für Wasserstoff, Fluor oder Chlor steht,

und

(A)

m für die Zahl 0 steht,

n für die Zahl 0 steht und

Z für Wasserstoff, für gegebenenfalls durch Fluor, Chlor, Cyano, Carboxy, $C_1$-$C_4$-Alkoxy-carbonyl, $C_1$-$C_4$-Alkoxy-carbonyl-$C_1$-$C_2$-alkoxy-carbonyl und/oder $C_1$-$C_4$-Alkylamino-carbonyl substituiertes $C_1$-$C_4$-Alkyl, für gegebenenfalls durch Fluor, Chlor, Cyano, Carboxy und/oder $C_1$-$C_4$-Alkoxy-carbonyl substituiertes $C_3$-$C_4$-Alkenyl oder für gegebenenfalls durch Fluor, Chlor und/oder Methyl substituiertes Benzyl steht, oder

(B)

m für die Zahl 2 steht,

n für die Zahlen 0 oder 1 steht,

Y für Sauerstoff oder eine der Gruppierungen -NH-, -NH-CO-, -NH-COO-, -NH-$SO_2$- steht, und

Z für Wasserstoff, für gegebenenfalls durch Fluor, Chlor, Cyano, Carboxy, $C_1$-$C_4$-Alkoxy-carbonyl und/oder $C_1$-$C_4$-Alkylamino-carbonyl substituiertes $C_1$-$C_4$-Alkyl, für gegebenenfalls durch Fluor, Chlor, Cyano, Carboxy und/oder $C_1$-$C_4$-Alkoxy-carbonyl substituiertes $C_3$-$C_4$-Alkenyl, für jeweils gegebenenfalls durch Fluor, Chlor, Brom, Nitro, Methyl, Ethyl, Trifluormethyl, Methoxy, Ethoxy, Difluormethoxy, Trifluormethoxy, Chlordifluormethoxy, Methylthio, Difluormethylthio, Trifluormethylthio, Chlordifluormethylthio, Methylsulfonyl, Dimethylaminosulfonyl, Methoxycarbonyl und/ oder Ethoxycarbonyl substituiertes Phenyl oder Benzyl steht, oder

(C)

m für die Zahl 2 steht,

n für die Zahl 0 steht und

Z für Chlor, für gegebenenfalls durch Chlor und/ oder $C_1$-$C_3$-Alkyl substituiertes Pyrazolyl oder für gegebenenfalls durch $C_1$-$C_3$-Alkyl substituiertes Imidazolyl steht.

**4.** Verfahren zur Herstellung von (Hetero)Aryloxynaphthalinen mit über Schwefel gebundenen Substituenten der allgemeinen Formel (I) gemäß Anspruch 1, dadurch gekennzeichnet, daß man

(a) für den Fall, daß m für 2 steht, n für 1 steht, Y für Sauerstoff steht, Z für Wasserstoff oder ein Metalläquivalent steht und $R^1$, $R^2$, $R^3$, $R^4$ und X die in Anspruch 1 angegebenen Bedeutungen haben,

Halogen-(hetero)aryl-Verbindungen der allgemeinen Formel (II)

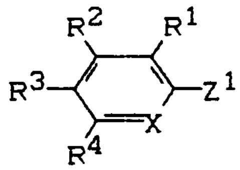

(II)

in welcher

$R^1$, $R^2$, $R^3$, $R^4$ und X die in Anspruch 1 angegebenen Bedeutungen haben und

$Z^1$ für Halogen steht,

mit Hydroxynaphthalinsulfonsäuren der allgemeinen Formel (III)

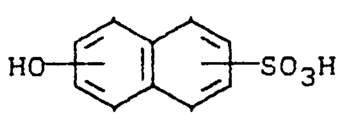

(III)

oder deren Metallsalzen

gegebenenfalls in Gegenwart eines Säureakzeptors und gegebenenfalls in Gegenwart eines Verdünnungsmittels umsetzt und gegebenenfalls anschließend mit einer starken Säure behandelt, oder daß man

(b) für den Fall, daß m für die Zahlen 0, 1 oder 2 steht, n für die Zahl 0 steht, Z für Halogen steht und $R^1$, $R^2$, $R^3$, $R^4$ und X die in Anspruch 1 angegebenen Bedeutungen haben,

Verbindungen der allgemeinen Formel (I), in welcher m für die Zahlen 0, 1 oder 2 steht, n für die

Zahl 1 steht, Y für Sauerstoff steht, Z für Wasserstoff oder ein Metalläquivalent steht und $R^1$, $R^2$, $R^3$, $R^4$ und X die in Anspruch 1 angegebenen Bedeutungen haben,
mit einem Halogenierungsmittel, gegebenenfalls in Gegenwart eines Katalysators bzw. Halogenierungshilfsmittels, und gegebenenfalls in Gegenwart eines Verdünnungsmittels umsetzt, oder daß man
(c) für den Fall, daß m für die Zahlen 0, 1 oder 2 steht, n für die Zahl 0 steht, Z für Wasserstoff oder ein Metalläquivalent steht und $R^1$, $R^2$, $R^3$, $R^4$ und X die in Anspruch 1 angegebenen Bedeutungen haben,
Verbindungen der allgemeinen Formel (I), in welcher m für die Zahl 2 steht, n für die Zahl 0 steht, Z für Halogen steht und $R^1$, $R^2$, $R^3$, $R^4$ und X die in Anspruch 1 angegebenen Bedeutungen haben,
mit einem Reduktionsmittel, gegebenenfalls in Gegenwart eines Reduktionshilfsmittels, und gegebenenfalls in Gegenwart eines Verdünnungsmittels umsetzt, oder daß man
(d) für den Fall, daß m für die Zahlen 0, 1 oder 2 steht, n für die Zahl 1 steht und $R^1$, $R^2$, $R^3$, $R^4$, X, Y und Z die in Anspruch 1 angegebenen Bedeutungen haben,
Verbindungen der Formel (I), in welcher m für die Zahlen 0, 1 oder 2 steht, n für die Zahl 0 steht, Z für Halogen steht und $R^1$, $R^2$, $R^3$, $R^4$ und X die in Anspruch 1 angegebenen Bedeutungen haben,
mit nucleophilen Verbindungen der allgemeinen Formel (IV)

$M\text{-}(Y)_n\text{-}Z$ (IV)

in welcher
n, Y und Z die in Anspruch 1 angegebenen Bedeutungen haben und
M für Wasserstoff oder ein Metalläquivalent steht,
gegebenenfalls in Gegenwart eines Säureakzeptors und gegebenenfalls in Gegenwart eines Verdünnungsmittels umsetzt, oder daß man
(e) für den Fall, daß Z mit Ausnahme von Wasserstoff und Halogen die in Anspruch 1 angegebene Bedeutung hat und m, n, $R^1$, $R^2$, $R^3$, $R^4$, X und Y die in Anspruch 1 angegebenen Bedeutungen haben,
Verbindungen der Formel (I) in welcher Y für Sauerstoff oder NH steht, Z für Wasserstoff steht und m, n, $R^1$, $R^2$, $R^3$, $R^4$ und X die in Anspruch 1 angegebenen Bedeutungen haben,
mit elektrophilen Verbindungen der allgemeinen Formel (V)

$Z^2\text{-}(Y^1)_n\text{-}Z^3$ (V)

in welcher
n für die Zahlen 0 oder 1 steht,
$Y^1$ für CO oder $SO_2$ steht,
$Z^2$ für Halogen steht und
$Z^3$ mit Ausnahme von Wasserstoff und Halogen die in Anspruch 1 für Z angegebene Bedeutung hat,
gegebenenfalls in Gegenwart eines Säureakzeptors und gegebenenfalls in Gegenwart eines Verdünnungsmittels umsetzt
und gegebenenfalls aus den nach den Verfahren (a) bis (e) hergestellten Verbindungen der Formel (I) Salze herstellt.

**5.** Herbizide Mittel, gekennzeichnet durch einen Gehalt an mindestens eines Verbindung der Formel (I) gemäß den Ansprüchen 1 bis 3.

**6.** Verfahren zur Bekämpfung von Unkräutern, dadurch gekennzeichnet, daß man Verbindungen der Formel (I) gemäß den Ansprüchen 1 bis 3 auf die Unkräuter und/oder ihren Lebensraum einwirken läßt.

**7.** Verwendung von Verbindungen der Formel (I) gemäß den Ansprüchen 1 bis 3 zur Bekämpfung von Unkräutern.

**8.** Verfahren zur Herstellung von herbiziden Mitteln, dadurch gekennzeichnet, daß man Verbindungen der Formel (I) gemäß den Ansprüchen 1 bis 3 mit Streckmitteln und/oder oberflächenaktiven Substanzen vermischt.

**9.** Verbindungen der Formel

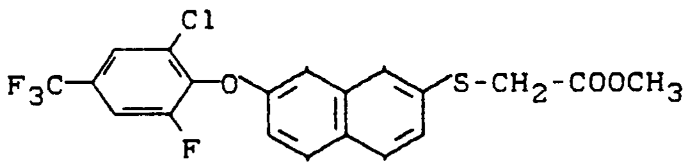

**10.** Die Verbindung der Formel

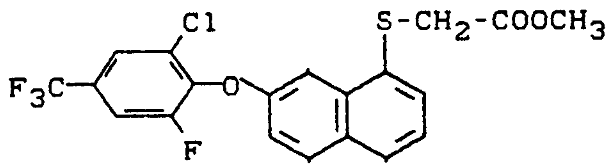

## Claims

**1.** (Hetero)aryloxynaphthalenes having substituents bonded via sulphur and of the general formula (I)

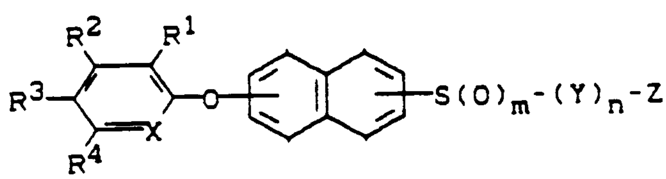

(I)

in which

- m represents the numbers 0, 1 or 2,
- n represents the numbers 0 or 1,
- $R^1$ represents hydrogen, halogen, cyano or trifluoromethyl,
- $R^2$ represents hydrogen or halogen,
- $R^3$ represents halogen, trifluoromethyl, trifluoromethoxy, trifluoromethylthio or trifluoromethylsulphonyl,
- $R^4$ represents hydrogen or halogen,
- X represents nitrogen or the grouping C-$R^5$, wherein
- $R^5$ represents hydrogen or halogen,
- Y represents oxygen or one of the groupings

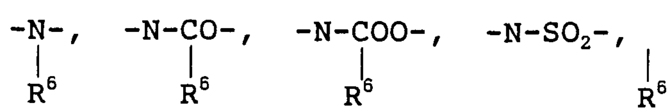

wherein

- $R^6$ represents hydrogen, alkyl, -CO-Z or -$SO_2$-Z and
- Z represents hydrogen, halogen or an optionally substituted radical from the series comprising alkyl, cycloalkyl, cycloalkylalkyl, alkenyl, alkinyl, aralkyl, aryl and heteroaryl,

and salts of compounds of the formula (I).

**2.** Compounds of the general formula (I) according to Claim 1, characterised in that in this formula

m represents the numbers 0, 1 or 2,
n represents the numbers 0 or 1,
$R^1$ represents hydrogen, fluorine, chlorine, bromine, cyano or trifluoromethyl,
$R^2$ represents hydrogen, fluorine or chlorine,
$R^3$ represents fluorine, chlorine, bromine, trifluoromethyl, trifluoromethoxy, trifluoromethylthio or trifluoromethylsulphonyl,
$R^4$ represents hydrogen, fluorine or chlorine,
X represents nigrogen or the grouping C-$R^5$, wherein
$R^5$ represents hydrogen, fluorine, chlorine or bromine,
Y represents oxygen or one of the groupings

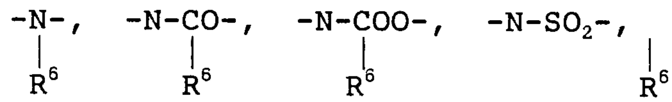

wherein
$R^6$ represents hydrogen, $C_1$-$C_4$-alkyl, -CO-Z or -$SO_2$-Z and
Z represents hydrogen or halogen, or $C_1$-$C_6$-alkyl which is optionally substituted by halogen, cyano, carboxyl, $C_1$-$C_4$-alkoxy, $C_1$-$C_4$-alkylcarbonyl, $C_1$-$C_4$-alkoxy-carbonyl, $C_1$-$C_4$-alkoxy-carbonyl-$C_1$-$C_4$-alkoxy-carbonyl and/or $C_1$-$C_4$-alkylamino-carbonyl, or $C_3$-$C_6$-cycloalkyl or $C_3$-$C_6$-cycloalkyl-$C_1$-$C_4$-alkyl, in each case optionally substituted by halogen and/or $C_1$-$C_4$-alkyl, or $C_2$-$C_6$-alkenyl or $C_2$-$C_6$-alkinyl, in each case optionally substituted by halogen, cyano, carboxyl and/or $C_1$-$C_4$-alkoxy-carbonyl, or phenyl, naphthyl, phenyl-$C_1$-$C_4$-alkyl or naphthyl-$C_1$-$C_4$-alkyl, in each case optionally substituted by halogen, cyano, carboxyl, nitro, $C_1$-$C_4$-alkyl, $C_1$-$C_2$-halogenoalkyl, $C_1$-$C_4$-alkoxy, $C_1$-$C_2$-halogenoalkoxy, $C_1$-$C_4$-alkylthio, $C_1$-$C_2$-halogenoalkylthio, $C_1$-$C_4$-alkylsulphinyl, $C_1$-$C_4$-alkylsulphonyl, di-($C_1$-$C_2$-alkyl)-aminosulphonyl, di-($C_1$-$C_2$-alkyl)-amino-carbonyl, di-($C_1$-$C_2$-alkyl)-amino, $C_1$-$C_4$-alkoxy-carbonyl and/or $C_1$-$C_2$-alkylenedioxy, or represents a heterocyclic radical from the series comprising pyrazolyl, imidazolyl and triazolyl, which is optionally substituted by halogen, $C_1$-$C_4$-alkyl, $C_1$-$C_2$-halogenoalkyl, $C_1$-$C_4$-alkoxy and/or $C_1$-$C_2$-halogenoalkoxy.

**3.** Compounds of the general formula (I) according to Claim 1, characterized in that, in this formula,
$R^1$ represents cyano, fluorine or chlorine,
$R^2$ represents hydrogen, fluorine or chlorine,
$R^3$ represents chlorine, trifluoromethyl or trifluoromethylsulphonyl,
$R^4$ represents hydrogen, fluorine or chlorine,
X represents nitrogen or the grouping C-$R^5$, wherein
$R^5$ represents hydrogen, fluorine or chlorine
and
(A)
m represents the number 0,
n represents the number 0, and
Z represents hydrogen, $C_1$-$C_4$-alkyl which is optionally substituted by fluorine, chlorine, cyano, carboxyl, $C_1$-$C_4$-alkoxy-carbonyl, $C_1$-$C_4$-alkoxy-carbonyl-$C_1$-$C_2$-alkoxy-carbonyl and/or $C_1$-$C_4$-alkylamino-carbonyl, or $C_3$-$C_4$-alkenyl which is optionally substituted by fluorine, chlorine, cyano, carboxyl and/or $C_1$-$C_4$-alkoxycarbonyl, or benzyl which is optionally substituted by fluorine, chlorine and/or methyl, or
(B)
m represents the number 2,
n represents the number 0 or 1,
Y represents oxygen or one of the groupings -NH-, -NH-CO-, -NH-COO- or -NH-$SO_2$- and
Z represents hydrogen, $C_1$-$C_4$-alkyl which is optionally substituted by fluorine, chlorine, cyano, carboxyl, $C_1$-$C_4$-alkoxy-carbonyl, and/or $C_1$-$C_4$-alkylamino-carbonyl, or $C_3$-$C_4$-alkenyl which is optionally substituted by fluorine, chlorine, cyano, carboxyl and/or $C_1$-$C_4$-alkoxycarbonyl, or phenyl or benzyl, in each case optionally substituted by fluorine, chlorine, bromine, nitro, methyl, ethyl, trifluoromethyl, methoxy, ethoxy, difluoromethoxy, trifluoromethoxy, chlorodifluoromethoxy, methylthio, difluoromethylthio, trifluoromethylthio,

chlorodifluoromethlylthio, methylsulphonyl, dimethylaminosulphonyl, mthoxycarbonyl and/or ethoxy-carbonyl, or

(C)

m represents the number 2,

n represents the number 0 and

Z represents chlorine, pyrazolyl which is optionally substituted by chlorine and/or $C_1$-$C_3$-alkyl, or imidazolyl which is optionally substituted by $C_1$-$C_3$-alkyl.

**4.** Process for the preparation of (hetero)aryloxynaphthalenes having substituents bonded via sulphur and of the general formula (I) according to Claim 1, characterized in that

(a) in the case where m represents 2, n represents 1, Y represents oxygen, Z represents hydrogen or a metal equivalent and $R^1$, $R^2$, $R^3$, $R^4$ and X have the meaning given in Claim 1,

halogeno-(hetero)aryl compounds of the general formula (II)

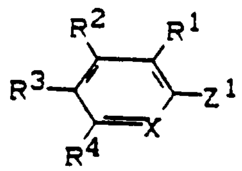

(II)

in which

$R^1$, $R^2$, $R^3$, $R^4$ and X have the meanings given in Claim 1, and

$Z^1$ represents halogen,

are reacted with hydroxynaphthalenesulphonic acids of the general formula (III)

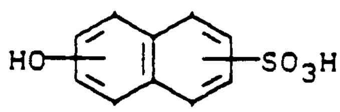

(III)

or metal salts thereof

if appropriate in the presence of an acid acceptor and if appropriate in the presence of a diluent, and, if appropriate, the products are then treated with a strong acid, or in that

(b) in the case where m represents the numbers 0, 1 or 2, n represents the number 0, Z represents halogen and $R^1$, $R^2$, $R^3$, $R^4$ and X have the meanings given in Claim 1,

compounds of the general formula (I) in which m represents the numbers 0, 1 or 2, n represents the number 1, Y represents oxygen, Z represents hydrogen or a metal equivalent and $R^1$, $R^2$, $R^3$, $R^4$ and X have the meanings given in Claim 1,

are reacted with a halogenating agent, if appropriate in the presence of a catalyst or halogenating auxiliary and if appropriate in the presence of a diluent, or in that

(c) in the case where m represents the numbers 0, 1 or 2, n represents the number 0, Z represents hydrogen or a metal equivalent and $R^1$, $R^2$, $R^3$, $R^4$ and X have the meanings given in Claim 1,

compounds of the general formula (I) in which m represents the number 2, n represents the number 0, Z represents halogen and $R^1$, $R^2$, $R^3$, $R^4$ and X have the meanings given in Claim 1,

are reacted with a reducing agent, if appropriate in the presence of a reduction auxiliary and if appropriate in the presence of a diluent, or in that

(d) in the case where m represents the numbers 0, 1 or 2, n represents the number 1 and $R^1$, $R^2$, $R^3$, $R^4$, X, Y and Z have the meanings given in Claim 1, compounds of the formula (I) in which m represents the numbers 0, 1 or 2, n represents the number 0, Z represents halogen and $R^1$, $R^2$, $R^3$, $R^4$ and X have the meanings given in Claim 1,

are reacted with nucleophilic compounds of the general formula (IV)

$M$-$(Y)_n$-$Z$ (IV)

in which

n, Y and Z have the meanings given in Claim 1, and
M represents hydrogen or a metal equivalent,

if appropriate in the presence of an acid acceptor and if appropriate in the presence of a diluent, or in that

(e) in the case where Z has the meanings given in Claim 1, with the exception of hydrogen and halogen, and m, n, $R^1$, $R^2$, $R^3$, $R^4$, X and Y have the meanings given in Claim 1,
compounds of the formula (I) in which Y represents oxygen or NH, Z represents hydrogen and m, n, $R^1$, $R^2$, $R^3$, $R^4$ and X have the meanings given in Claim 1, are reacted with electrophilic compounds of the general formula (V)

$Z^2-(Y^1)_n-Z^3$ (V)

in which
n represents the numbers 0 or 1,
$Y^1$ represents CO or $SO_2$,
$Z^2$ represents halogen and
$Z^3$ has the meaning given in Claim 1, for Z, with the exception of hydrogen and halogen,

if appropriate in the presence of an acid acceptor and if appropriate in the presence of a diluent,

and, if appropriate, salts are prepared by customary methods from the compounds of the formula (I) prepared by the processes described above.

**5.** Herbicidal agents, characterized in that they contain at least one compound of the formula (I) according to Claims 1 to 3.

**6.** Method of combating weeds, characterized in that compounds of the formula (I) according to Claims 1 to 3 are allowed to act on the weeds and/or their environment.

**7.** Use of compounds of the formula (I) according to Claims 1 to 3 for combating weeds.

**8.** Process for the preparation of herbicidal agents, characterized in that compounds of the formula (I) according to Claims 1 to 3 are mixed with extenders and/or surface-active substances.

**9.** The compounds of the formula

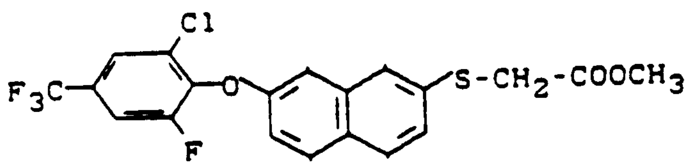

**10.** The compound of the formula

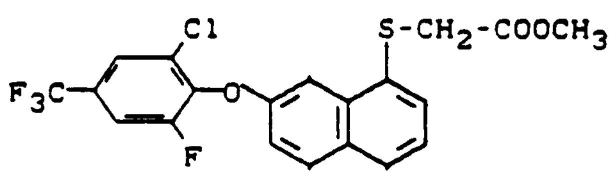

## Revendications

**1.** (Hétéro)aryloxynaphtalènes comportant des substituants liés par l'intermédiaire d'un atome de soufre et répondant à la formule générale (I)

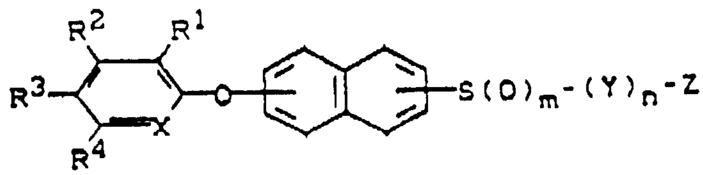 (I)

dans laquelle

m représente les nombres 0, 1 ou 2,

n représente le nombre 0 ou 1,

$R^1$ représente un atome d'hydrogène, un atome d'halogène, un groupe cyano ou un groupe trifluorométhyle,

$R^2$ représente un atome d'hydrogène ou un atome d'halogène,

$R^3$ représente un atome d'halogène, un groupe trifluorométhyle, un groupe trifluorométhoxy, un groupe trifluorométhylthio ou un groupe trifluorométhylsulfonyle,

$R^4$ représente un atome d'hydrogène ou un atome d'halogène,

X représente un atome d'azote ou le groupement C-$R^5$, où

$R^5$ représente un atome d'hydrogène ou un atome d'halogène,

Y représente un atome d'oxygène ou un des groupements

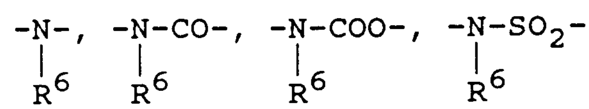

ou

$R^6$ représente un atome d'hydrogène, un groupe alkyle, -CO-Z ou -$SO_2$-Z et

Z représente un atome d'hydrogène, un atome d'halogène ou un radical éventuellement substitué choisi dans la série comprenant un radical alkyle, un radical cycloalkyle, un radical cycloalkylalkyle, un radical alcényle, un radical alcynyle, un radical aralkyle, un radical aryle et un radical hétéroaryle,

ainsi que des sels des composés de formule (I).

2. Composés de formule générale (I) selon la revendication 1, qui se caractérise par le fait que, dans ces composés,

m représente les nombres 0, 1 ou 2,

n représente le nombre 0 ou 1,

$R^1$ représente un atome d'hydrogène, un atome de fluor, un atome de chlore, un atome de brome, un groupe cyano ou un groupe trifluorométhyle,

$R^2$ représente un atome d'hydrogène, un atome de fluor ou un atome de chlore,

$R^3$ représente un atome de fluor, un atome de chlore, un atome de brome, un groupe trifluorométhyle, un groupe trifluorométhoxy, un groupe trifluorométhylthio ou un groupe trifluorométhylsulfonyle,

$R^4$ représente un atome d'hydrogène, un atome de fluor ou un atome de chlore,

X représente un atome d'azote ou le groupement C-$R^5$, où

$R^5$ représente un atome d'hydrogène, un atome de fluor, un atome de chlore ou un atome de brome,

Y représente un atome d'oxygène ou un des groupements

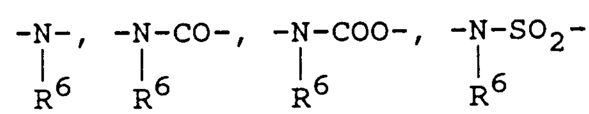

où

$R^6$ représente un atome d'hydrogène, un groupe alkyle en $C_1$-$C_4$, -CO-Z ou -$SO_2$-Z et

Z représente un atome d'hydrogène, un atome d'halogène, un groupe alkyle en $C_1$-$C_6$ éventuellement substitué par un atome d'halogène, par un groupe cyano, par un groupe carboxyle, par un groupe alcoxy en $C_1$-$C_4$, par un groupe alkyl(en $C_1$-$C_4$)carbonyle, par un groupe alcoxy(en $C_1$-$C_4$)carbonyle, par un groupe alcoxy(en $C_1$-$C_4$)carbonylalcoxy(en $C_1$-$C_4$)-carbonyle et/ou par un groupe alkyl(en $C_1$-$C_4$)aminocarbonyle, un groupe cycloalkyle en $C_3$-$C_6$ ou bien un groupe cycloalkyl(en $C_3$-$C_6$)alkyle en $C_1$-$C_4$, chacun étant éventuellement substitué par un atome d'halogène et/ou par un groupe alkyle en $C_1$-$C_4$, un groupe alcényle en $C_2$-$C_6$ ou un groupe alcynyle en $C_2$-$C_6$, chacun étant éventuellement substitué par un atome d'halogène, par un groupe cyano, par un groupe carboxyle et/ou par un groupe alcoxy(en $C_1$-$C_4$)carbonyle, un groupe phényle, un groupe naphtyle, un groupe phénylalkyle en $C_1$-$C_4$ ou un groupe naphtylalkyle en $C_1$-$C_4$, chacun étant éventuellement substitué par un atome d'halogène, par un groupe cyano, par un groupe carboxyle, par un groupe nitro, par un groupe alkyle en $C_1$-$C_4$, par un groupe halogénalkyle en $C_1$-$C_2$, par un groupe alcoxy en $C_1$-$C_4$, par un groupe halogénalcoxy en $C_1$-$C_2$, par un groupe alkyl(en $C_1$-$C_4$)thio, par un groupe halogénalkyl(en $C_1$-$C_2$)thio, par un groupe alkyl(en $C_1$-$C_4$)sulfinyle, par un groupe alkyl(en $C_1$-$C_4$)sulfonyle, par un groupe di-(alkyl en $C_1$-$C_2$)aminosulfonyle, par un groupe di-(alkyl en $C_1$-$C_2$)aminocarbonyle, par un groupe di-(alkyl en $C_1$-$C_2$)amino, par un groupe alcoxy(en $C_1$-$C_4$)-carbonyle et/ou par un groupe alkylène(en $C_1$-$C_2$)dioxy, ou encore un hétérocycle choisi dans la série comprenant un groupe pyrazolyle, un groupe imidazolyle, un groupe triazolyle éventuellement substitué par un atome d'halogène, par un groupe alkyle en $C_1$-$C_4$, par un groupe halogénalkyle en $C_1$-$C_2$, par un groupe alcoxy en $C_1$-$C_4$ et/ou par un groupe halogénalcoxy en $C_1$-$C_2$.

**3.** Composés de formule générale (I) selon la revendication 1, qui se caractérise par le fait que, dans ces composés,

$R^1$ représente un groupe cyano, un atome de fluor ou un atome de chlore,

$R^2$ représente un atome d'hydrogène, un atome de fluor ou un atome de chlore,

$R^3$ représente un atome de chlore, un groupe trifluorométhyle ou un groupe trifluorométhylsulfonyle,

$R^4$ représente un atome d'hydrogène, un atome de fluor ou un atome de chlore,

X représente un atome d'azote ou le groupement C-$R^5$, où

$R^5$ représente un atome d'hydrogène, un atome de fluor ou un atome de chlore,

et

(A)

m représente le nombre 0,

n représente le nombre 0 et

Z représente un atome d'hydrogène, un groupe alkyle en $C_1$-$C_4$ éventuellement substitué par un atome de fluor, par un atome de chlore, par un groupe cyano, par un groupe carboxyle, par un groupe alcoxy(en $C_1$-$C_4$)carbonyle, par un groupe alcoxy(en $C_1$-$C_4$)carbonylalcoxy-(en $C_1$-$C_2$)carbonyle et/ou par un groupe alkyl(en $C_1$-$C_4$)aminocarbonyle, un groupe alcényle en $C_3$-$C_4$ éventuellement substitué par un atome de fluor, par un atome de chlore, par un groupe cyano, par un groupe carboxyle et/ou par un groupe alcoxy(en $C_1$-$C_4$)-carbonyle ou bien un groupe benzyle éventuellement substitué par un atome de fluor, par un atome de chlore et/ou par un groupe méthyle, ou

(B)

m représente le nombre 2,

n représente le nombre 0 ou 1,

Y représente un atome d'oxygène ou un des groupements -NH-, -NH-CO-, -NH-COO-, -NH-$SO_2$- et

Z représente un atome d'hydrogène, un groupe alkyle en $C_1$-$C_4$ éventuellement substitué par un atome de fluor, par un atome de chlore, par un groupe cyano, par un groupe carboxyle, par un groupe alcoxy(en $C_1$-$C_4$)carbonyle et/ou par un groupe alkyl(en $C_1$-$C_4$)-aminocarbonyle, un groupe alcényle en $C_3$-$C_4$ éventuellement substitué par un atome de fluor, par un atome de chlore, par un groupe cyano, par un groupe carboxyle et/ou par un groupe alcoxy(en $C_1$-$C_4$)carbonyle, un groupe phényle ou un groupe benzyle, chacun étant éventuellement substitué par un atome de fluor, par un atome de chlore, par un atome de brome, par un groupe nitro, par un groupe méthyle, par un groupe éthyle, par un groupe

trifluorométhyle, par un groupe méthoxy, par un groupe éthoxy, par un groupe difluorométhoxy, par un groupe trifluorométhoxy, par un groupe chlorodifluorométhoxy, par un groupe méthylthio, par un groupe difluorométhylthio, par un groupe trifluorométhylthio, par un groupe chlorodifluorométhylthio, par un groupe méthylsulfonyle, par un groupe diméthylaminosulfonyle, par un groupe méthoxycarbonyle et/ou par un groupe éthoxycarbonyle, ou

(C)

m représente le nombre 2,

n représente le nombre 0 et

Z représente un atome de chlore, un groupe pyrazolyle éventuellement substitué par un atome de chlore et/ou par un groupe alkyle en $C_1$-$C_3$, ou bien un groupe imidazolyle éventuellement substitué par un groupe alkyle en $C_1$-$C_3$.

**4.** Procédé pour la préparation d'(hétéro)aryloxynaphtalènes comportant des substituants liés par l'intermédiaire d'un atome de soufre et répondant à la formule générale (I) selon la revendication 1, caractérisé en ce que

(a) pour le cas où m représente 2, n représente 1, Y représente un atome d'oxygène, Z représente un atome d'hydrogène ou un équivalent métallique et $R^1$, $R^2$, $R^3$, $R^4$ et X ont les significations indiquées dans la revendication 1,

on fait réagir des composés halogéno-(hétéro)aryle répondant à la formule générale (II)

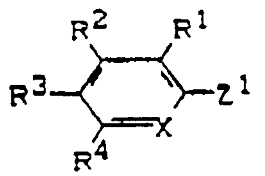

(II)

dans laquelle

$R^1$, $R^2$, $R^3$, $R^4$ et X ont les significations indiquées à la revendication 1 et

$Z^1$ représente un atome d'halogène,

avec des acides hydroxynaphtalènesulfoniques répondant à la formule générale (III)

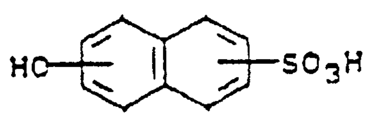

(III)

ou avec des sels métalliques de ces derniers,

éventuellement en présence d'un accepteur d'acides et éventuellement en présence d'un diluant, et ensuite on les traite éventuellement avec un acide fort, ou bien en ce que

(b) dans le cas où m représente les nombres 0, 1 ou 2, n représente le nombre 0, Z représente un atome d'halogène et $R^1$, $R^2$, $R^3$, $R^4$ et X ont les significations indiquées à la revendication 1,

on fait réagir des composés répondant à la formule générale (I) dans laquelle m représente les nombres 0, 1 ou 2, n représente le nombre 1, Y représente un atome d'oxygène, Z représente un atome d'hydrogène ou un équivalent métallique et $R^1$, $R^2$, $R^3$, $R^4$ et X ont les significations indiquées dans la revendication 1,

avec un agent d'halogénation, éventuellement en présence d'un catalyseur ou d'un adjuvant d'halogénation et éventuellement en présence d'un diluant, ou bien en ce que

(c) dans le cas où m représente les nombres 0, 1 ou 2, n représente le nombre 0, Z représente un atome d'hydrogène ou un équivalent métallique et $R^1$, $R^2$, $R^3$, $R^4$ et X ont les significations indiquées à la revendication 1,

on fait réagir des composés répondant à la formule générale (I) dans laquelle m représente le nombre 2, n représente le nombre 0, Z représente un atome d'halogène et $R^1$, $R^2$, $R^3$, $R^4$ et X ont les significations indiquées dans la revendication 1,

avec un agent de réduction, éventuellement en présence d'un adjuvant de réduction et éventuellement en présence d'un diluant, ou bien en ce que

(d) dans le cas où m représente les nombres 0, 1 ou 2, n représente le nombre 1 et $R^1$, $R^2$, $R^3$, $R^4$, X, Y et Z ont les significations indiquées à la revendication 1,
on fait réagir des composés répondant de formule (I) dans laquelle m représente les nombres 0, 1 ou 2, n représente le nombre 0, Z représente un atome d'halogène et $R^1$, $R^2$, $R^3$, $R^4$ et X ont les significations indiquées dans la revendication 1,
avec des composés nucléophiles de formule générale (IV)

$M\text{-}(Y)_n\text{-}Z$ (IV)

dans laquelle
n, Y et Z ont les significations indiquées à la revendication 1 et
M représente un atome d'hydrogène ou un équivalent métallique,
éventuellement en présence d'un accepteur d'acides et éventuellement en présence d'un diluant, ou bien en ce que
(e) dans le cas où Z, à l'exception d'un atome d'hydrogène et d'un atome d'halogène, a la signification indiquée à la revendication 1 et m, n $R^1$, $R^2$, $R^3$, $R^4$, X et Y ont les significations indiquées à la revendication 1,
on fait réagir des composés de formule (I) dans laquelle Y représente un atome d'oxygène ou NH, Z représente un atome d'hydrogène et m, n, $R^1$, $R^2$, $R^3$, $R^4$ et X ont les significations indiquées dans la revendication 1,
avec des composés électrophiles de formule générale (V)

$Z^2\text{-}(Y^1)_n\text{-}Z^3$ (V)

dans laquelle
n représente le nombre 0 ou 1,
$Y^1$ représente CO ou $SO_2$,
$Z^2$ représente un atome d'halogène et
$Z^3$ à l'exception d'un atome d'hydrogène et d'un atome d'halogène, a la signification indiquée à la revendication 1 pour Z,
éventuellement en présence d'un accepteur d'acides et éventuellement en présence d'un diluant,
et on prépare éventuellement des sels à partir des composés de formule (I) préparés conformément aux procédés (a) à (e).

5. Agents herbicides caractérisés par une teneur en au moins un composé de formule (I) selon les revendications 1 à 3.

6. Procédé pour lutter contre les mauvaises herbes, caractérisé en ce qu'on laisse agir des composés de formule (I) selon les revendications 1 à 3, sur les mauvaises herbes et/ou sur leur biotope.

7. Utilisation de composés de formule (I) selon les revendications 1 à 3, pour lutter contre les mauvaises herbes.

8. Procédé pour la préparation d'agents herbicides, caractérisé en ce qu'on mélange des composés de formule (I) selon les revendications 1 à 3, avec des diluants et/ou des substances tensioactives.

9. Composés de formule

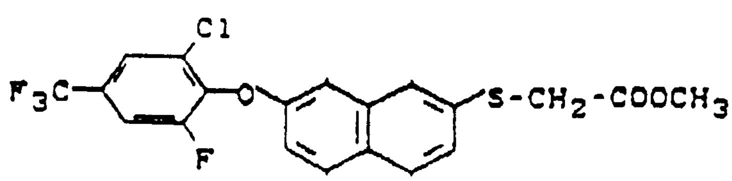

10. Composé de formule